# EUROPEAN PATENT SPECIFICATION

(11) **EP 4 225 803 B1**
(45) Date of publication and mention of the grant of the patent: **15.04.2026**
(21) Application number: 21798092.9
(22) Date of filing: 11.10.2021
(51) Int. Cl.: C07K 17/08, B82Y 5/00, A61P 35/04, A61K 9/00, A61K 9/51, A61K 38/00

(54) **NANOPARTICLE FOR ANTI-CANCER PEPTIDES AND USES THEREOF**
NANOPARTIKEL FÜR ANTIKREBSPEPTIDE UND VERWENDUNGEN DAVON
NANOPARTICULE POUR PEPTIDES ANTICANCÉREUX ET SES UTILISATIONS

(30) Priority: 09.10.2020 GB 202016022
(43) Date of publication of application: 16.08.2023
(73) Proprietor: King's College London, London WC2R 2LS (GB)
(72) Inventor: ULMSCHNEIDER, Martin Bernhard, London SE1 1DB (GB); CHEN, Charles Huang, London SE1 0NZ (GB); HU, Che-Ming Jack, Taipei, 11529 (TW); LIU, Yu-Han, Taipei, 11529 (TW)
(74) Representative: Viering, Jentschura & Partner mbB Patent- und Rechtsanwälte
(86) International application number: PCT/GB2021/052621
(87) International publication number: WO 2022/074402

(56) References cited:
- WO-A1-2018/165655
- WO-A1-2019/023622
- KR-A- 20170 015 715
- CHARLES H. CHEN ET AL: "Simulation-Guided Rational de Novo Design of a Small Pore-Forming Antimicrobial Peptide", JOURNAL OF THE AMERICAN CHEMICAL SOCIETY, vol. 141, no. 12, 6 March 2019 (2019-03-06), pages 4839 - 4848, XP055765551, ISSN: 0002-7863, DOI: 10.1021/jacs.8b11939

## Description

### Field of the Invention

The invention relates to a nanoparticle for anti-cancer peptides (ACPs) which can be used in the treatment of cancer.

### Background to the Invention

Tumours are heterogeneous at the cellular level, consisting of a range of different subtypes of cancer cells. Among these subtypes, cancer stem cells (CSCs) are increasingly recognised as a major difficulty in traditional pharmaceutical treatment using current anti-cancer drugs. Breast cancer is the second most common cancer around the world, and mostly occurs in women. Several studies have shown that breast cancer stem cells might develop resistance to conventional anti-cancer drugs to survive, self-renew, differentiate and relapse.¹⁻⁶ CSCs readily evolve resistance to anti-cancer drugs and the chemotherapeutic treatment of solid tumours typically results in a significant increase in the share of drug-resistant CSCs in the patient. This can lead to relapse and the formation of metastases. Furthermore, it is possible that breast tumours can be different within the same patient and conventional anti-cancer drugs may fail.⁷⁻⁹ Treatment with higher doses is difficult as commonly used anti-cancer drugs, such as doxorubicin, have a generally high toxicity towards healthy tissues, resulting in acute damage to organs such as the liver, kidneys, and heart.¹⁰⁻¹² Therefore, there is an urgent, unmet need to develop new anti-cancer drugs that have improved selectivity towards cancer cells, leaving healthy tissues unharmed at doses that are sufficient to kill all bulk cancer and CSCs in a solid tumour.

Membrane-lytic peptides present promising anti-cancer treatment alternatives to chemotherapeutics, which are typically cytotoxic to non-cancerous cells and tend to induce drug resistance via stress-induced mutagenesis¹⁻⁶. Despite continued research efforts on the therapeutic utility of membrane-lytic peptides for antimicrobial applications¹³⁻¹⁵, delivery methods for their success as anti-cancer therapeutics need to be improved upon. Here, the present inventors demonstrate the efficient encapsulation of potent, selective, anti-cancer peptides in nanoparticles which possess high tolerability and lead to effective tumour growth inhibition or eradication. It is demonstrated that the nanoparticles significantly improve the anticancer activity of the cargo peptide while simultaneously reducing its toxicity. This is unlike small molecule nanoparticles such as Doxil^{®}, the FDA approved carrier for doxorubicin, which whilst reducing side effects, does not improve the overall anti-cancer efficacy of the chemotherapeutic cargo (doxorubicin).

WO 2018/165655 A1 relates to a methodology combining MD simulations and database-guided high-throughput screening to rationally design pore forming membrane-active peptides.

WO 2019/023622 A1 relates to nanoparticles comprising a defined set of biologically active agents that provide for targeted stimulation of non-responsive T cells, e.g. T cells involved in cancer.

KR 2017/0015715 A relates to uses of leucine-rich repeats and immunoglobulin-like domains 2 (LRIG2) for diagnosing or treating cancer and, more specifically, to a pharmaceutical composition for preventing or treating endometrial cancer comprising an LRIG2 gene or a protein which the gene codes as effective components, to a biomarker for diagnosing endometrial cancer, to a composition for diagnosing endometrial cancer, to a kit for diagnosing endometrial cancer and to a screening method of a medicine for endometrial cancer.

The work of the present inventors demonstrates a novel delivery system for membrane-lytic peptides, opening up a new channel in the race against cancer.

### Summary of the Invention

In a first aspect of the invention, there is provided a nanoparticle comprising one or more peptides having a sequence comprising the motif GLLxLLxLLLxAAG, wherein each x is independently selected from arginine (R), histidine (H), lysine (K), aspartic acid (D) or glutamic acid (E), wherein the nanoparticle has a diameter of 1 to 200 nm and wherein the motif is selected from SEQ ID NO: 2, SEQ ID NO: 4, SEQ ID NO: 6, SEQ ID NO: 14, SEQ ID NO: 25 or SEQ ID NO: 26 and mixtures thereof.

The inventors have surprisingly found that a family of peptides conforming to the claimed formula have improved selectivity towards cancer cells, leaving healthy tissues unharmed at doses that are sufficient to kill all bulk cancer and CSCs in a solid tumour. Unlike many conventional anti-cancer drugs, the pore-forming membrane-active peptides developed here target and disrupt the plasma membrane to kill cancer cells. This removes the complication of having to transport the drug into the cytoplasm and as such, the peptides have improved tumour penetration in comparison to traditional chemotherapy agents. The presently claimed peptides act by selectively targeting the plasma membranes of cancer cells and forming pores therein, thus killing the cells by short-circuiting their electrochemical gradient. Without wishing to be bound by theory, it is thought that the peptides directly target the lipid composition and chemical microenvironment of the cancer cell membrane. Consequently, the peptides are far less likely to induce resistance (in a similar way that it is difficult for cells to develop resistance to detergents) as it is difficult for the tumour cells to modify their lipid composition.¹⁶⁻¹⁸

Several of the disclosed peptides have nano-molar activity against bulk cancer and CSCs, comparable to current approved anti-cancer drugs such as salinomycin. Furthermore, in one of the best current *in vitro* breast cancer models, the mammosphere model, which mimics a real solid tumour by growing cells into a spherical clump, several of the peptides disclosed herein exhibit superior activity against cancer cells, while retaining reduced toxicity towards normal, healthy cells.

The peptides work in both the L and D amino acid forms (the latter being a major advantage for *in vivo* stability against protease degradation) to selectively eliminate two-dimensionally grown cancer cells, as well as three-dimensional (spheroid) cancer cell cultures at very low micromolar, and in some cases, nanomolar concentrations. 3 to >200-fold higher concentrations are required to harm non-cancerous human breast and kidney cells.

The peptides are inexpensive and straightforward to synthesize, are easy to modify and high-throughput screen, and offer a chemical and structural repertoire to target cancer cells specifically.

The presently claimed peptides are *de novo* designed, and have no known natural analogues, as confirmed by comparison with extant peptide databases. Short flexible peptides of this type will have low immunogenicity and are thus suitable for pharmaceutical applications.

As herein described the term "peptide" refers to any peptide comprising amino acids joined to each other by peptide bonds or modified peptide bonds, *i.e.,* peptide isosteres. The peptide generally will contain naturally occurring amino acids, but may include amino acid sequences modified either by natural processes, such as post-translational processing, or by chemical modification techniques, which are well known in the art. Such modifications are well described in basic texts. Modifications can occur anywhere in a peptide, including the peptide backbone, the amino acid side-chains and the amino or carboxyl termini. It will be appreciated that the same type of modification may be present in the same or varying degrees at several sites in a given peptide. Also, a given peptide may contain many types of modifications.

Preferably, the peptides are isolated peptides. The term *"isolated"* means that the peptide is removed from its original environment. For example, a peptide present in a living animal is not isolated, but the same peptide, or a fragment of such a peptide, separated from some or all of the coexisting materials in the natural system, is isolated. Such peptides could be part of a vector and/or peptides could be part of a composition, and still be isolated in that such vector or composition is not part of its natural environment.

The one or more peptides are loaded onto or into a nanoparticle. Preferably the nanoparticle comprises a plurality of peptides. It has been found that delivering peptides using a nanoparticle produces surprisingly good results. It is thought that the localised delivery provided by the nanoparticles allows the peptides to form pores in the cell membranes of cancer cells more effectively and thereby brings about more efficient killing of the cancer cells.

The term nanoparticle refers to a particle that is about 1 to about 200 nm in diameter. Preferably the nanoparticle is from about 5 to about 100 nm, about 10 to about 50 nm, and even more preferably about 20 nm in diameter.

Peptide loading into the nanoparticle can be carried out be either adsorption or encapsulation. Such techniques are well known to one skilled in the art.

Preferably the nanoparticle is biodegradable. Preferably the nanoparticle is polymeric. Suitable polymers that may form some of the disclosed nanoparticles may include, but are not limited to, biodegradable α-hydroxy polyesters and biocompatible polyethers. In some aspects, exemplary polyesters include, for example, PLA, PLGA, PEG, PEG, PEGylated polymers and copolymers of lactide and glycolide (e.g., PEGylated PLA, PEGylated PGA, PEGylated PLGA), and derivatives thereof. In other aspects, suitable polymers include, for example, poly anhydrides, poly(ortho ester) PEGylated poly(ortho ester), poly (caprolactone), PEGylated poly(caprolactone), polylysine, PEGylated poiylysine, poly(ethylene inline), PEGylated polyethylene imine), poly(L-lactide-co-L-lysine), poly(serine ester), poly(4-hydroxy-L-proline ester), poly[a-(4-aminobutyl)-L-glycolic acid], and combinations and derivatives thereof. In a particularly preferred embodiment, the nanoparticle comprises polyethyleneglycol methyl ether polylactide-co-glycolide (PEG-PLGA).

In other aspects, a polymer matrix may comprise one or more acrylic polymers. Exemplary acrylic polymers include, for example, acrylic acid and methacrylic acid copolymers, methyl methacrylate copolymers, ethoxy ethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid polyacrylamide) copolymer, aminoalkyl methacrylate copolymer, glycidyl methacrylate copolymers, polycyanoacrylates, and combinations thereof. The matrix may include dextran, acylated dextran, chitosan (e.g., acetylated to various levels), poly(vinyl) alcohol (for example, hydrolyzed to various degrees), and/or alginate, e.g. alginate complexed to bivalent cations such as a calcium alginate complex.

Nanoparticles disclosed herein may include one, two, three or more biocompatible and/or biodegradable polymers. For example, a contemplated nanoparticle may include about 10 to about 99 weight percent of one or more block co-polymers that include a biodegradable polymer and polyethylene glycol, and about 0 to about 50 weight percent of a biodegradable homopolymer. Exemplar}' nanoparticles may include about 40 to about 90 weight percent poly(lactic) acid-poly(ethylene)glycol copolymer or about 40 to about 80 weight percent poly(lactic) acid-poly(ethylene)glycol copolymer. Such poly(lactic) acid-block-poly(ethylene)glycol copolymer may include poly(lactic acid) having a number average molecular weight of about 5 to 100 kDa, and poly(ethylene)glycol having a number average molecular weight of about 2 to about 10 kDa, for example, about 4 to about 6 kDa. For example, a disclosed therapeutic nanoparticle may include about 70 to about 99 weight percent PLA-PEG and about 1 to about 25 weight percent active agent (i.e. the disclosed peptides), or about 30 to about 50 weight percent PLA-PEG, about 30 to about 50 weight percent PLA or PLGA, and about 5 to about 25 weight percent active agent. Such PLA ((poly)lactic acid) may have a number average molecular weight of about 5 to about 10 kDa. Such PLGA (poly lactic-co-glycolic acid) may have a number average molecular weight of about 8 to about 12 kDa. It should be appreciated that disclosed PLA.-PEG copolymers may include a chemical linker, oligomer, or polymer chain between the PLA and PEG blocks, e.g., may include PLA-linker-PEG.

Most preferably, the nanoparticles of the present invention comprise about 99 weight percent of poly(ethylene glycol) methyl ether-block-poly(lactide-co-glycolide) (PEG-PLGA, PEG average Mₐ 5,000, PLGA Mₙ 7,000) and 1 weight percent of the active ingredient (i.e. AGP).

Alternatively, a disclosed nanoparticle, which may have slow release properties, may include about 42 to about 45 weight percent PLA-PEG (with e.g. PIA about 16 kDa and PEG about 5 kDa), (e.g. 43.25% PLA-PEG), about 42 to 45 weight percent PL.A (e.g. about 75 kDa) (e.g. 43.25% PLA/75 kDa) and about 1 to 15 weight percent active agent. For example, disclosed nanoparticles may optionally include about 1 to about 50 weight percent poly(lactic) acid or poly(lactic) acid-co-poly(glycolic) acid (which does not include PEG, e.g a homopolymer of

PLA), or may optionally include about 1 to about 50 weight percent or about 10 to about 50 weight percent or about 30 to about 50 weight percent poly(lactic) acid or poly(lactic) acid-co-poly(glycolic) acid. In an embodiment, disclosed nanoparticles may include two polymers, e.g. PLA-PEG and PLA, in a weight ratio of about 30:60 to about 60:30, e.g, about 40:60, about 60:40, or about 50:50.

Such substantially homopolymeric poly(lactic) or poly(lactic)-co-poly (glycolic) acid may have a weight average molecular weight of about 2 to about 130 kDa, for example, about 20 to about 30 kDa, or about 100 to about 130 kDa.. Such homopolymeric PLA may have a number average molecule weight of about 5 to about 90 kDa, or about 5 to about 12 kDa, about 15 to about 30 kDa, or about 60 to about 90 kDa. Exemplary' homopolymeric PLA may have a number average molecular weight of about 8 kDa or a weight average molecular weight of about 12 kDa.

In certain aspects, disclosed nanoparticles may be optimized with a specific density of targeting moieties on the nanoparticle surface, such that e.g., an effective amount of targeting moiety is associated with the nanoparticle for delivery of the peptide. For example, the fraction of the biodegradable and/or biocompatible polymer matrix functionalized with a targeting moiety may be less than 80% of the total. According to another embodiment, the fraction of the biodegradable and/or biocompatible polymer matrix functionalized with a targeting moiety is less than about 50% of the total. Increased density of the targeting moiety may, in some embodiments, increase target binding (cell binding/target uptake).

Exemplary targeting moieties include, for example, proteins, peptides, antibodies, antibody fragments, saccharides, carbohydrates, glycans, cytokines, chemokines, nucleotides, lectins, lipids, receptors, steroids, neurotransmitters and combinations thereof. The choice of a. marker may vary- depending on the selected target, but in general, markers that may be useful in embodiments of the invention include, but are not limited to, ceil surface markers, a cancer antigen (CA), a glycoprotein antigen, a melanoma associated antigen (MAA), a proteolytic enzyme, an angiogenesis marker, a prostate membrane specific antigen (PMSA), a small cell lung carcinoma antigen (SCLCA), a hormone receptor, a tumour suppressor gene antigen, a cell cycle regulator antigen, a proliferation marker, and a human carcinoma antigen. Exemplary' targeting moieties include: 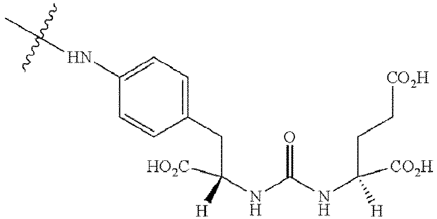 or 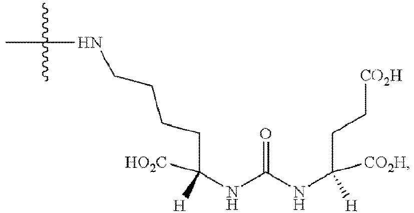 -lys-(urea)glu, which may be conjugated to PEG, e.g. a disclosed nanoparticle may include PLA-PEG-targeting moiety, e.g. S, S-2-{3-[1-carboxy-5-amino-pentyl]-ureido}-pentanedioic acid.

The one or more peptides may have a length of up to 50 amino acids. In some embodiments, the peptides have a length of up to 40 amino acids. In further embodiments, the peptides have a length of up to 30 amino acids. In various embodiments, the peptides have a length of up to 25 amino acids. In certain embodiments, the peptides have a length of up to 20 amino acids. In a number of embodiments, the peptides have a length of up to 18 amino acids. In some embodiments, the peptides have a length of up to 16 amino acids. In further embodiments, the peptides have a length of up to 1.5 amino acids. In certain embodiments, the peptides have a length of 14 amino acids.

The one or more peptides are preferably neutral or are anionic. Peptides which are neutral or anionic have been found to work very effectively which is surprising as anti-cancer peptides are traditionally cationic as it is thought that the positively charged cationic peptides would interact more effectively with the negatively charged phospholipid bilayer of cell membranes.

Outside the subject-matter of the claims, the peptide may comprise a sequence selected from any one of SEQ ID NO: 1, 3, 5, 6 to 13, 15 to 24, or 27 to 36 or mixtures thereof. Further not encompassed by the claims is an embodiment, where the peptide may consist of the sequence of any one of SEQ ID NO: 1, 3, 5, 6 to 13, 15 to 24, or 27 to 36.

In one embodiment, the nanoparticle comprises a peptide having a sequence comprising the motif GLLxLLELLLxAAG. The inventors have surprisingly found that peptides with this sequence have a better selectivity for cancer cells.

Outside the subject-matter of the claims, the sequence does not comprise SEQ ID NO: 29 or SEQ ID NO: 33.

According to the invention, the nanoparticle comprises a peptide having a sequence selected from SEQ ID NO: 2, SEQ ID NO: 4, SEQ ID NO: 6, SEQ ID NO: 14, SEQ ID NO: 25 or SEQ ID NO: 26 and mixtures thereof. More preferably, the pharmaceutically acceptable composition comprises a sequence selected from SEQ ID NO: 2, SEQ ID NO: 4, SEQ ID NO: 14, SEQ ID NO: 25 or SEQ ID NO: 26 and mixtures thereof. Even more preferably, the pharmaceutically acceptable composition comprises a sequence selected from SEQ ID NO: 25 and/or SEQ ID NO: 26. The inventors have found that these sequences have a particularly selective for cancer cells.

In one embodiment, the nanoparticle comprises a peptide wherein the peptide sequence consists of the motif GLLxLLxLLLxAAG.

The peptides can be present in either the D or the L form. In one embodiment, the nanoparticle comprises a peptide in the L form. It has been surprisingly found by the inventors that the peptides presented here are more selective for cancer cells when in the L form.

In one embodiment, the nanoparticle comprises a peptide which forms an alpha helical assembly. Preferably the peptide forms a pore in a cancer cell membrane. It is believed that the peptides directly target the lipid composition and chemical microenvironment of the cancer cell membrane and form pores therein that kill the cancer cells by short-circuiting their electrochemical gradient.

The N- and C-termini of the peptide sequence or motif may be any termini known to one skilled in the art and may include NH2, NH3⁺, COOH and COO⁻ for example.

A second aspect of the invention relates to a pharmaceutically acceptable composition comprising the nanoparticle as described above, and one or more pharmaceutically acceptable excipients, for use in the treatment of cancer.

It may be feasible in the scope of the invention that the pharmaceutically acceptable composition comprising the nanoparticle as described above, and one or more pharmaceutically acceptable excipients, are used for the manufacture of a medicament for the treatment of cancer.

The pharmaceutical composition may comprise a plurality of the nanoparticles as disclosed above.

The pharmaceutically acceptable composition of the present invention may be used to treat any type of cancer such as skin cancer, lung cancer, breast cancer, prostate cancer, colorectal cancer, bladder cancer, lymphomas, kidney cancer, pancreatic cancer or endometrial cancer. However, in a particular embodiment of the invention, the cancer is breast cancer.

The pharmaceutical composition comprising the nanoparticle may be for human or animal usage in human and veterinary medicine and will typically comprise one or more suitable excipients. Acceptable excipients for therapeutic use are well known in the pharmaceutical art, and are described, for example, in Remington's Pharmaceutical Sciences, Mack Publishing Co. (A. R. Gennaro edit. 1985). The choice of pharmaceutical excipient can be selected with regard to the intended route of administration and standard pharmaceutical practice. The pharmaceutical compositions may comprise as, or in addition to, the excipient, any suitable binder, lubricant, suspending agent, coating agent or solubilising agent.

Preservatives, stabilizers and dyes may be provided in the pharmaceutical composition. Examples of preservatives include sodium benzoate, sorbic acid and esters of p-hydroxybenzoic acid. Antioxidants and suspending agents may be also used.

The pharmaceutical composition may also comprise tolerance-promoting adjuvants and/or tolerance promoting cells. Tolerance promoting adjuvants include IL-10, recombinant cholera toxin B-subunit (rCTB), ligands for Toll-like receptor 2, as well as biologics and monoclonal antibodies that modulate immune responses, such as anti-CD3 and co-stimulation blockers, which may be co-administered with the peptide. Tolerance promoting cells include immature dendritic cells and dendritic cells treated with vitamin D3, (1alpha,25-dihydroxy vitamin D3) or its analogues.

When cancer is *"treated",* this means that one or more clinical manifestations of cancer are ameliorated. It does not mean that the symptoms of cancer are completely remedied so that they are no longer present in the patient, although in some methods, this may be the case. *"Treatment"* results in one or more of the symptoms of cancer being less severe than before treatment. For example, a tumour may be reduced in size or eradicated entirely.

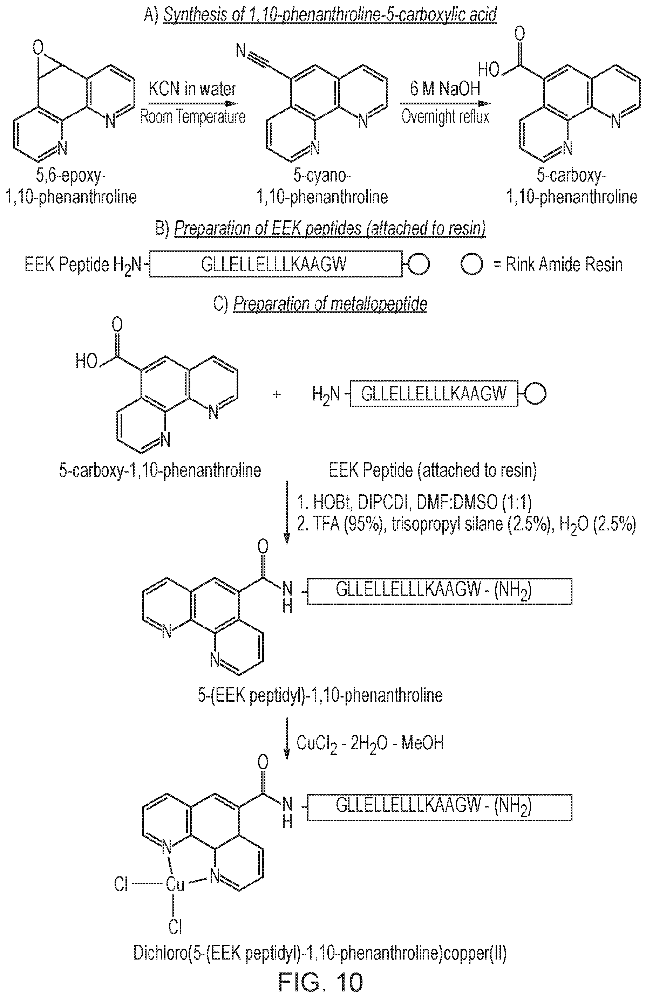

In one embodiment of the present invention, the composition is for use in combination with a chemotherapy agent. The inventors have found that due to the pore forming properties of the presently claimed peptides, this grants easier access to the target cancer cells for standard chemotherapeutic agents. The chemotherapeutic agent may be selected from cyclophosphamide, methotrexate, 5-fluorouracil, vinorelbine, doxorubicin, docetaxel, bleomycin, vinblastine, dacarbazine, mustine, vincristine, procarbazine, prednisolone, etoposide, cisplatin, epirubicin, methotrexate, capecitabine, vinorelbine, folinic acid, oxaliplatin and mixtures thereof. Preferably the chemotherapeutic agent is doxorubicin. One example of a means to conjugate the present peptides to a chemotherapeutic agent is provided in Figure 10.

There may be different composition/formulation requirements for the pharmaceutical composition dependent on the chosen delivery system. By way of example, the pharmaceutical composition of the present invention may be formulated to be delivered parenterally in which the composition is formulated in an injectable form, for delivery, by, for example, an intravenous, intradermal, intramuscular, subcutaneous or intraperitoneal route. For parenteral administration, the compositions may be best used in the form of a sterile aqueous solution which may contain other substances, for example enough salts or monosaccharides to make the solution isotonic with blood. Intradermal administration routes include any dermal-access means, for example, using microneedle-based injection and infusion systems (or other means to accurately target the intradermal space), needleless or needle-free ballistic injection of fluids or powders into the intradermal space, Mantoux-type intradermal injection, enhanced iontophoresis through microdevices, and direct deposition of fluid, solids, or other dosing forms into the skin, including the use of patches to deposit the composition onto the skin. The composition may also be formulated to be administered by oral or topical routes, including nasally, orally or epicutaneously. Preferably the composition is formulated to be delivered by an intravenous route.

The amount or dose of the pharmaceutical composition that is administered should be sufficient to effectively target cancer cells *in vivo.* The dose will be determined by the efficacy of the particular formulation and the location of the tumour in the subject, as well as the body weight of the subject to be treated.

The dose of the pharmaceutical composition will also be determined by the existence, nature, and extent of any adverse side effects that might accompany the administration of a particular formulation. Typically, a physician will decide the dosage of the peptides with which to treat each individual subject, taking into consideration a variety of factors, such as age, body weight, general health, diet, sex, compound/formulation to be administered, route of administration, and the severity of the condition being treated. The appropriate dosage can be determined by one skilled in the art. By way of non-limiting example, the total dose of the anti-cancer peptides of the present invention can be about 0.001 to about 1000 mg/kg body weight of the subject being treated, from about 0.01 to about 100 mg/kg body weight, from about 0.1 mg/kg to about 10 mg/kg, and from about 0.5 mg to about 5 mg/kg body weight. In another embodiment, the total dose of the peptides can be at a concentration from about 1 nM to about 10,000 nM, preferably from about 10 nM to about 5,000 nM, more preferably from about 100 nM to about 500 nM.

In a preferred embodiment, the composition comprising the nanoparticle of the present invention is administered at least once per month, preferably once every 1 to 4 weeks for four administrations.

Not claimed by the invention is a method of treatment of cancer in which the pharmaceutically acceptable composition of the invention is administered to a patient with cancer. In one embodiment the cancer is breast cancer. The references to methods of treatment by therapy or surgery or *in vivo* diagnosis methods in the examples of this description are to be interpreted as references to compounds, pharmaceutical compositions and medicaments of the present invention for use in those methods.

Not encompassed by the wording of the claims is a kit for treating cancer comprising the pharmaceutically acceptable composition of the invention, wherein the kit may be used for treating breast cancer. The kit may further comprise a chemotherapeutic agent.

### Detailed Description of the Invention

The invention will now be described in detail by way of example only with reference to the figures in which:

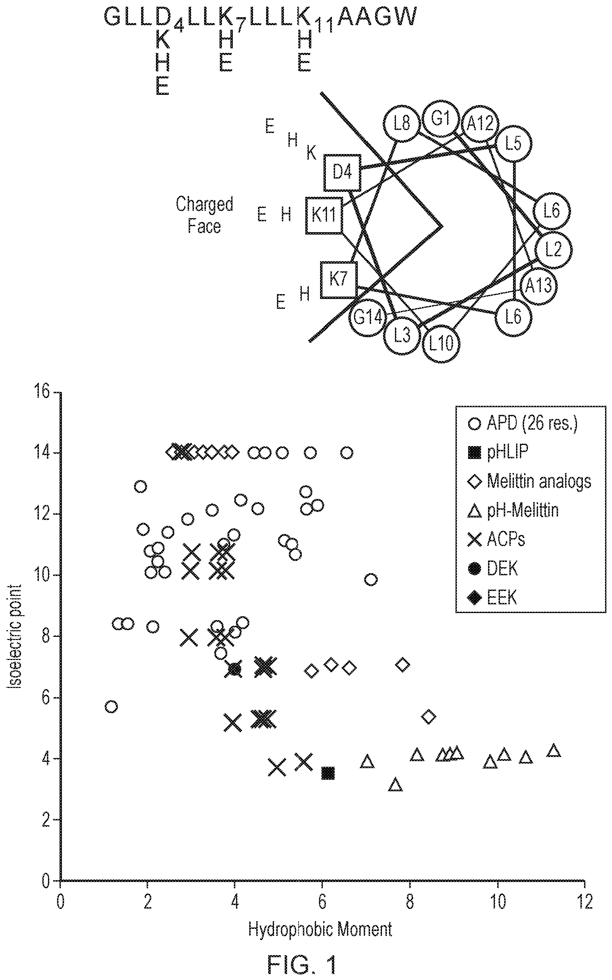

Figure 1 shows the design of a combinatorial leucine-rich peptide library and comparison with other pore-forming and cancer targeting membrane active peptides. A) Combinatorial peptide library sequences are shown together with their projection onto a helical wheel, which is the presumed membrane-active conformation. B) Comparison of the isoelectric point and hydrophobicity of the library peptides to other pore forming and cancer-targeting membrane-active peptides. Peptides that contain 26 amino acids in the antimicrobial peptide database (APD), melittin and its analogs (gain-of-function and loss-of-function analogs), pH-dependent melittin, and the cancer targeting pH-low insertion peptide (pHLIP).

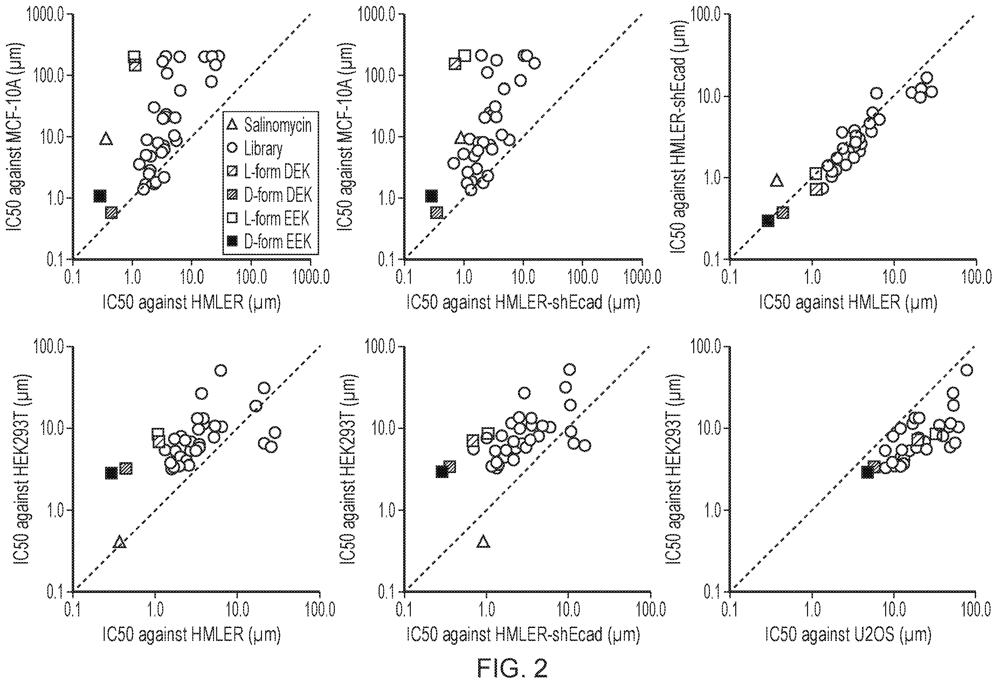

Figure 2 shows the results of an *in vitro* cytotoxicity screen of the library of the presently identified sequences, consisting of 36 combinatorial peptides (SEQ ID NO: 1 to 36) against different human cell lines, derived from both cancerous and healthy human tissues. Also shown are *in vitro* cytotoxicity screening results for selected D-form peptides, as well as the clinically used anti-cancer drugs salinomycin and doxorubicin. Cytotoxicity was evaluated for different human cell lines and is quantified using the half maximal inhibitory concentration (IC₅₀) for: A) HMLER versus MCF-10A, B) HMLER-shEcad versus MCF-10A, C) HMLER versus HMLER-shEcad, D) HMLER versus HEK293T, E) HMLER-shEcad versus HEK293T, and F) U2OS versus HEK293T.

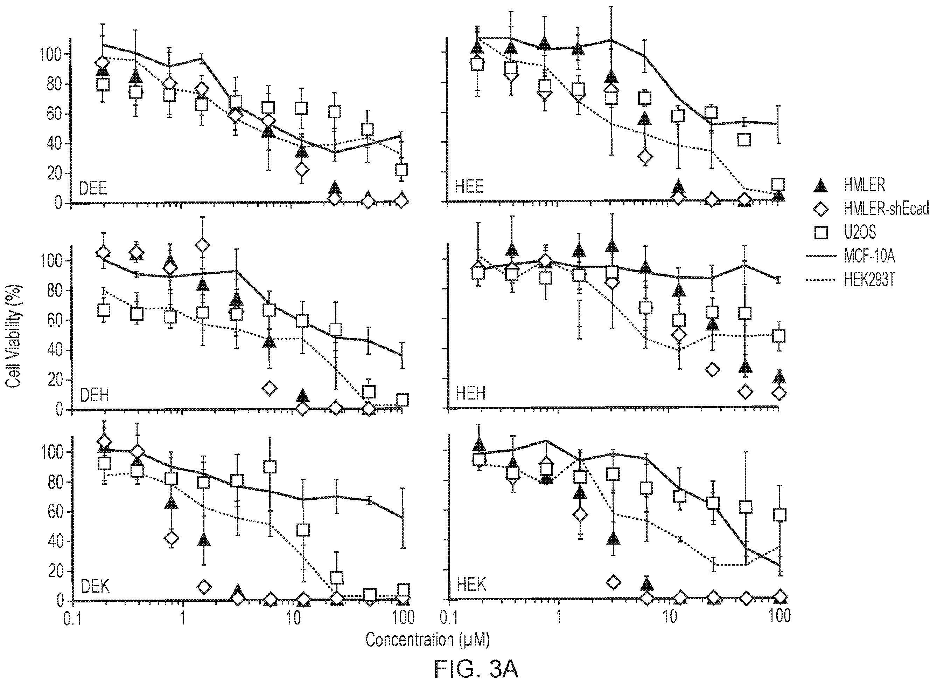

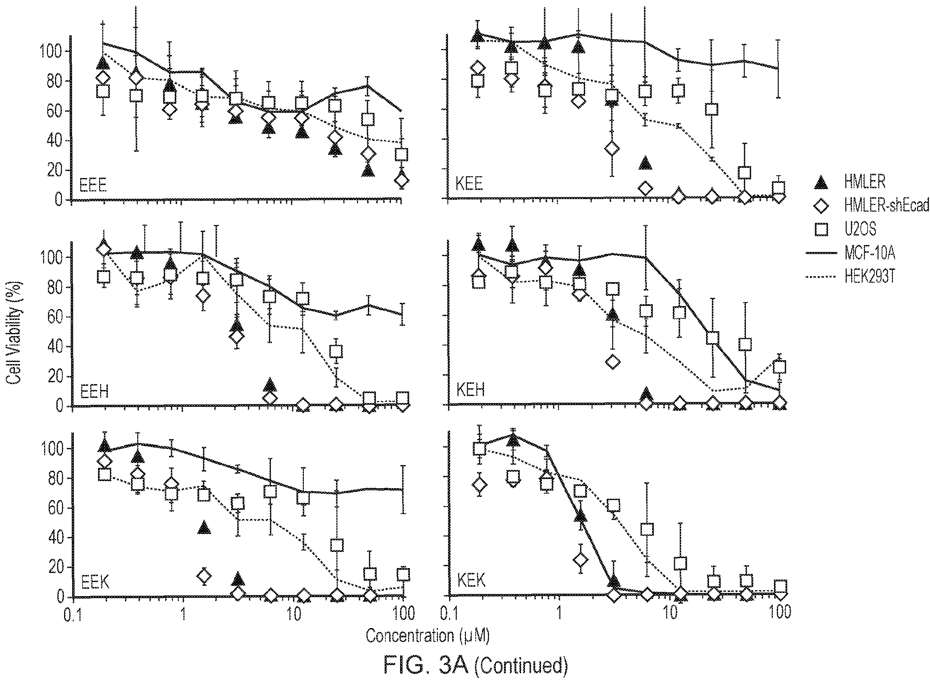

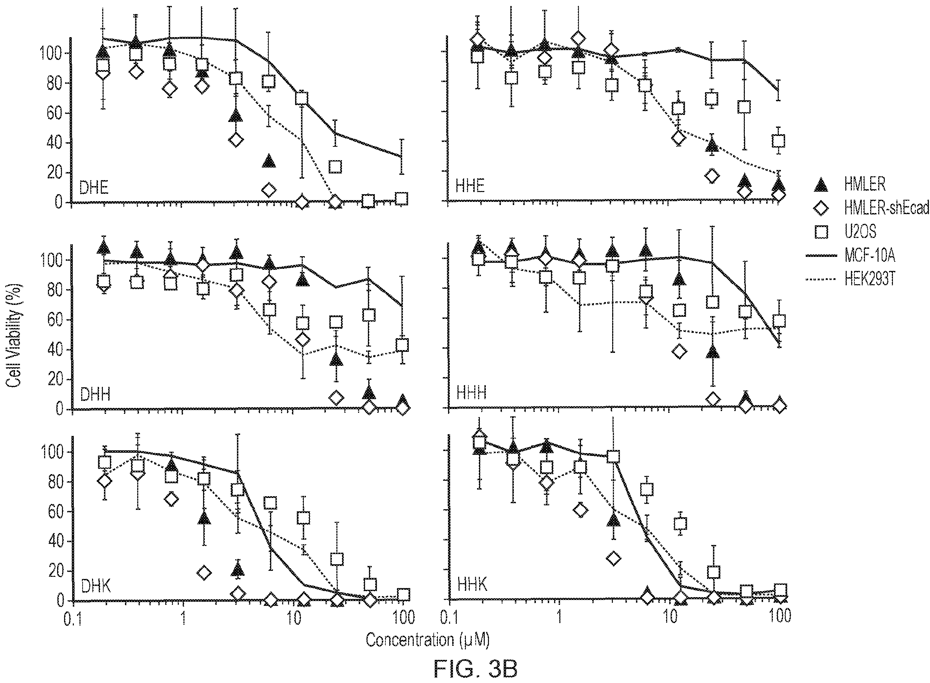

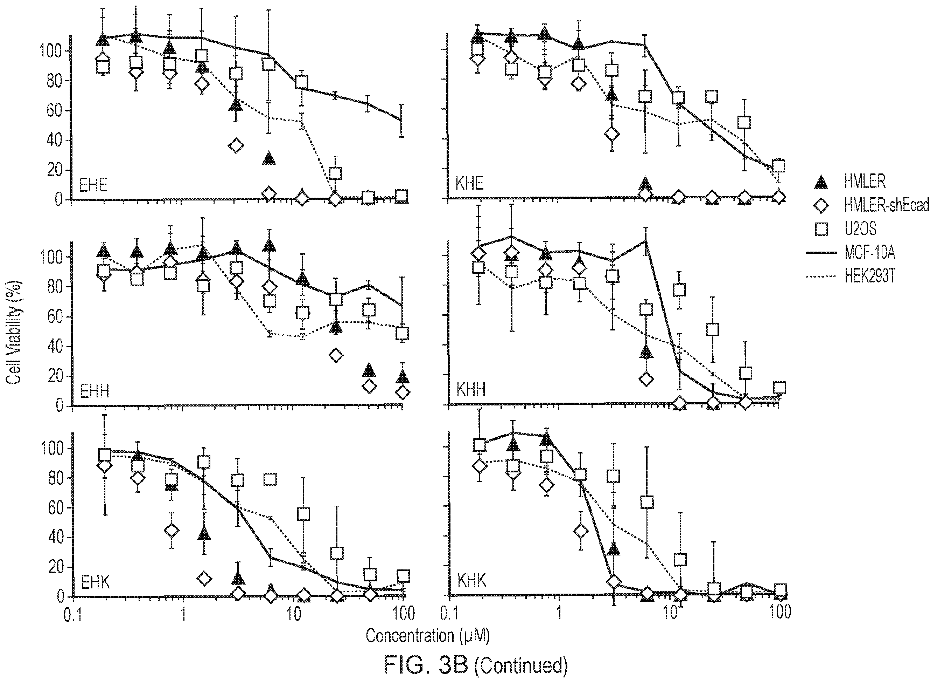

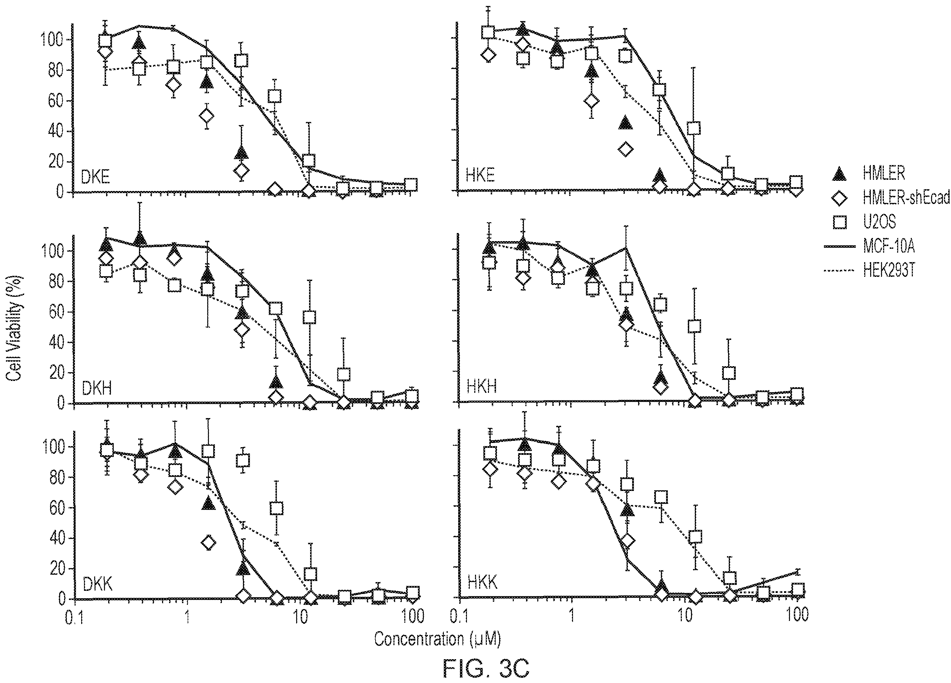

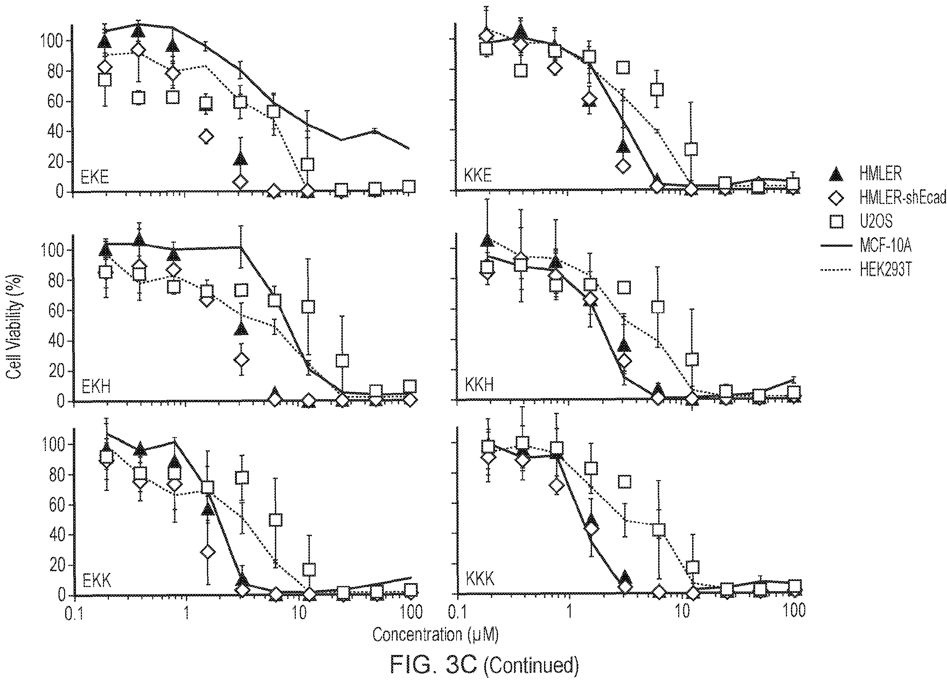

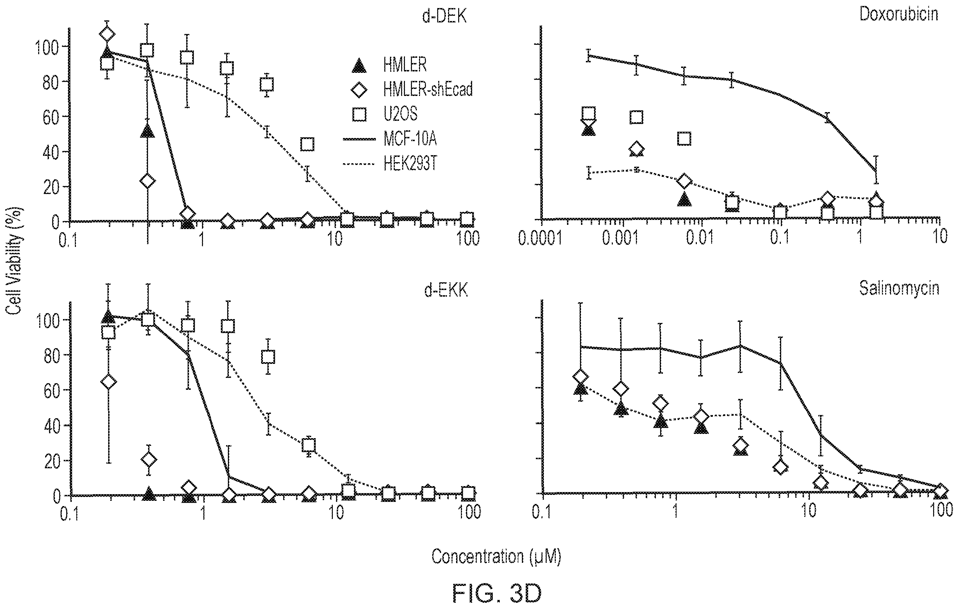

Figure 3 shows the *in vitro* cytotoxic dose response of two clinically used anti-cancer drugs doxorubicin and salinomycin, in comparison to two selected D-form anti-cancer peptides (D-form DEK, and D-form EEK), and 36 leucine-rich anti-cancer peptides against different human cell lines, e.g. HMLER (triangles), HMLER-shEcad (diamonds), MCF-10A (solid lines), U2OS (squares), and HEK293T (dotted lines).

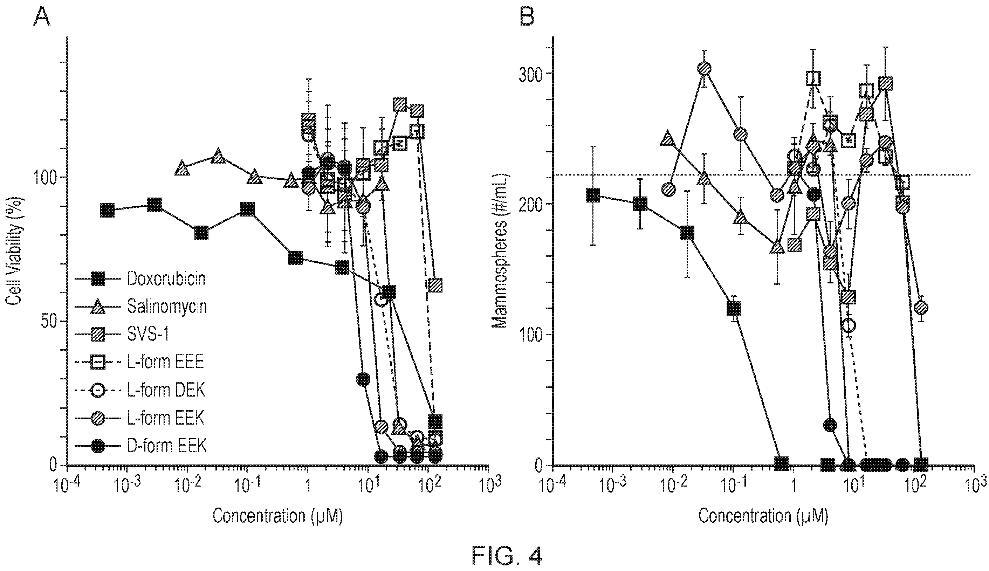

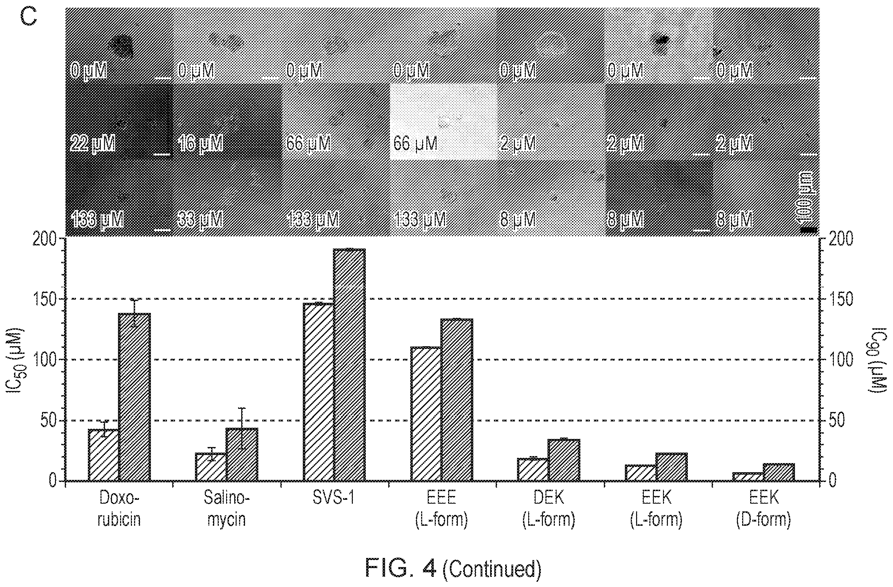

Figure 4 shows the tumoursphere (HMLER-shEcad cells) *in vitro* cytotoxicity and dose response of doxorubicin (filled squares), salinomycin (filled triangles) and the leucine-rich-based anti-cancer peptides L-form EEE (squares), L-form DEK (circles), L-form EEK (grey circles) and D-form EEK (black circles). A) Cell viability is measured to quantify the potency of the anti-cancer drugs against tumour cell (HMLER-shEcad) mammospheres. B) Mammosphere population after treatment with the selected anti-cancer compounds. The dashed line presents the expected negative control without any treatment. C) The measured IC₅₀ (grey bar) and IC₉₀ (black bar) of each anti-cancer drug and optical microscope images of the mammospheres at specific concentration. The scale bar is 100 µm.

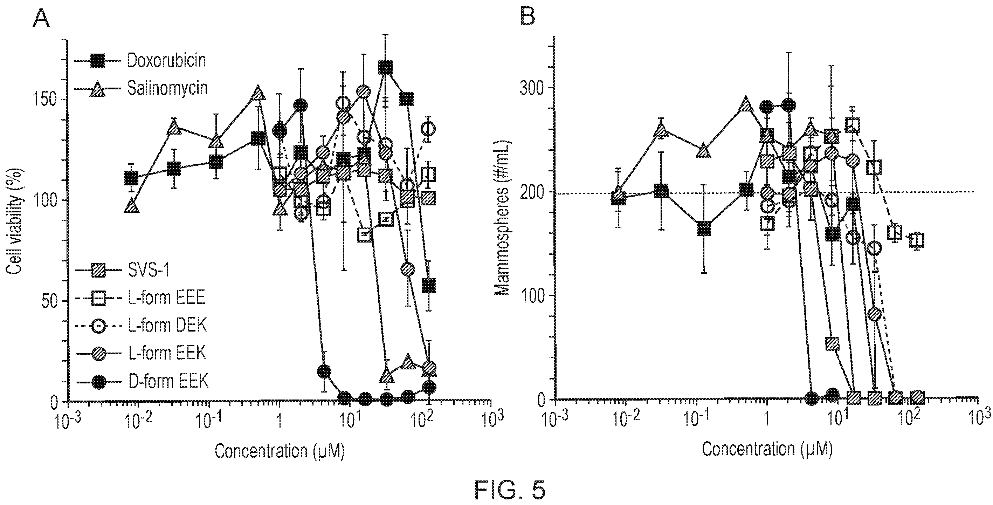

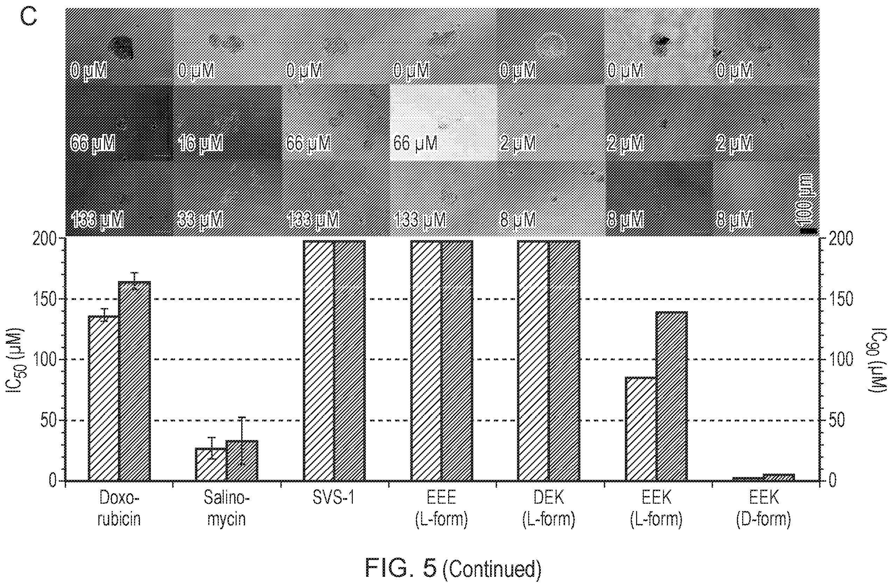

Figure 5 shows the mammosphere (MCA-10A cells) *in vitro* cytotoxicity and dose response of doxorubicin (filled squares), salinomycin (filled triangles) and the leucine-rich-based anti-cancer peptides L-form EEE (squares), L-form DEK (circles), L-form EEK (grey circles) and D-form EEK (black circles). Cell viability is measured to quantify the potency of the anti-cancer drugs against healthy human breast endothelial cell (MCA-10A) mammospheres. B) Mammosphere population after treatment with the selected anti-cancer compounds. The dashed line presents the negative control without any treatment. C) The measured IC₅₀ (solid bar) and IC₉₀ (bar) of each anti-cancer drug and optical microscope images of the mammospheres at specific concentration. The scale bar is 100 µm.

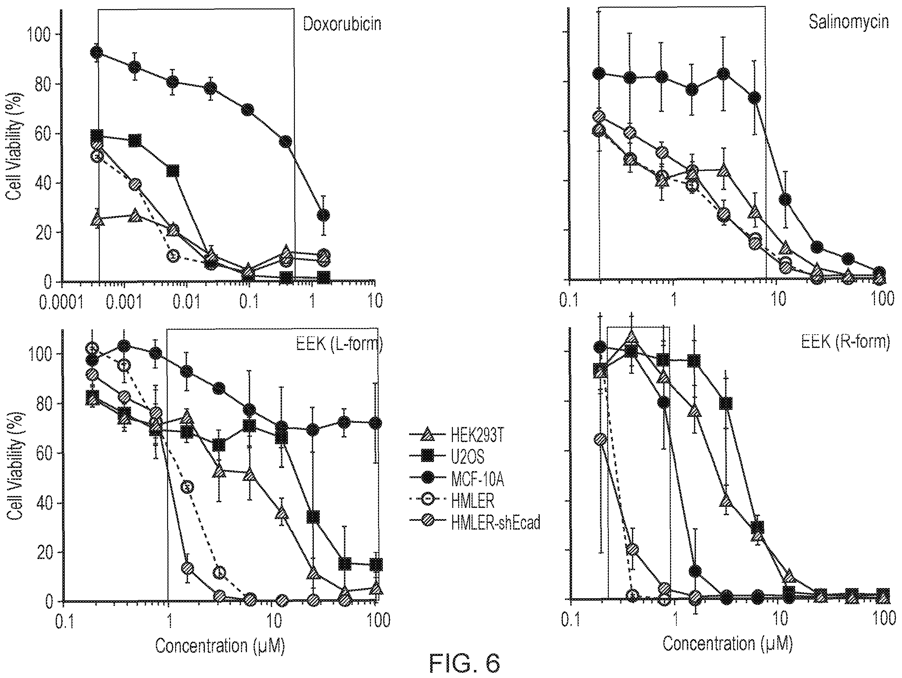

Figure 6 shows the *in vitro* cytotoxicity and dose response of doxorubicin, salinomycin, L-form EEK, and D-form EEK against different human cell lines: HMLER (circles), HMLER-shEcad (grey filled circles), U2OS (squares), MCF-10A (black filled circles), and HEK293T (triangles). The shaded regions indicate the ideal compound concentrations that have cell-selectivity towards cancer cell lines with less effect on normal cell lines (MCF-10A and HEK293T).

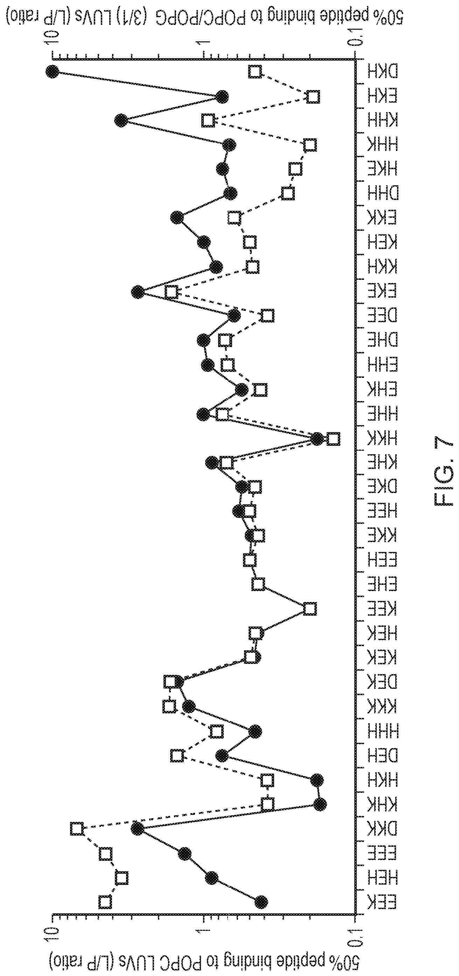

Figure 7 shows the results of the tryptophan fluorescence binding assay. It shows the lipid concentration at which 50% of the peptide binds to either a single lipid species POPC liposome (circles), or mixed lipid species POPC:POPG (ratio 3:1, squares) liposomes. In brief, 50 µM peptides were fixed and incubated with titrated POPC vesicles (black) or 3POPC/1POPG vesicles (grey) at concentrations of 0, 12.5, 25, 50, 100, 250, 500, 1000, 2500, and 5000 µM in phosphate buffered saline (IX, pH 7.4). The lipid concentration that causes 50% peptide binding was determined using a tryptophan fluorescent binding assay and the values are shown as lipid per peptide. This data demonstrates that the peptides of the invention can distinguish between a neutral vesicle (POPC) and a charged one (POPC/POPG), the latter acting as a model for a cancer cell (Warburg effect).

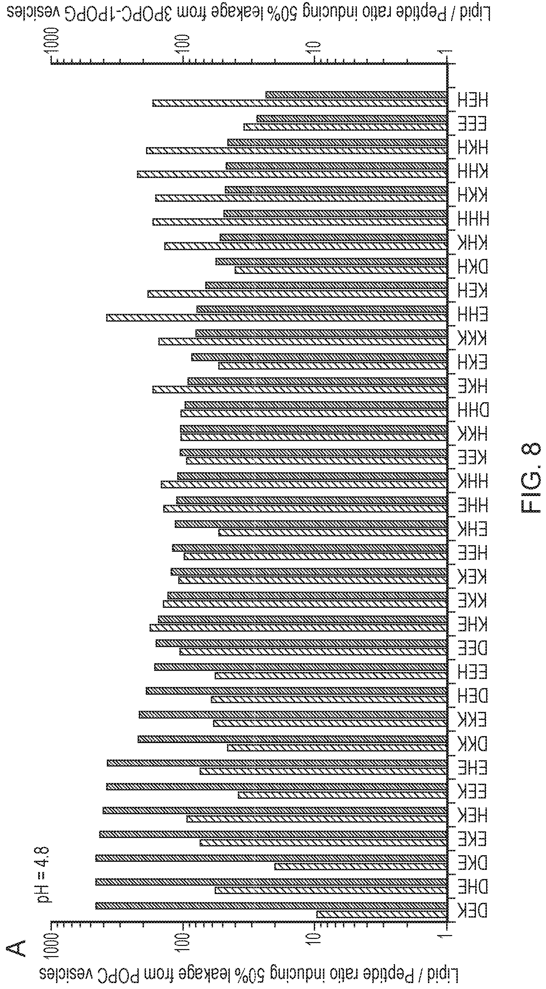

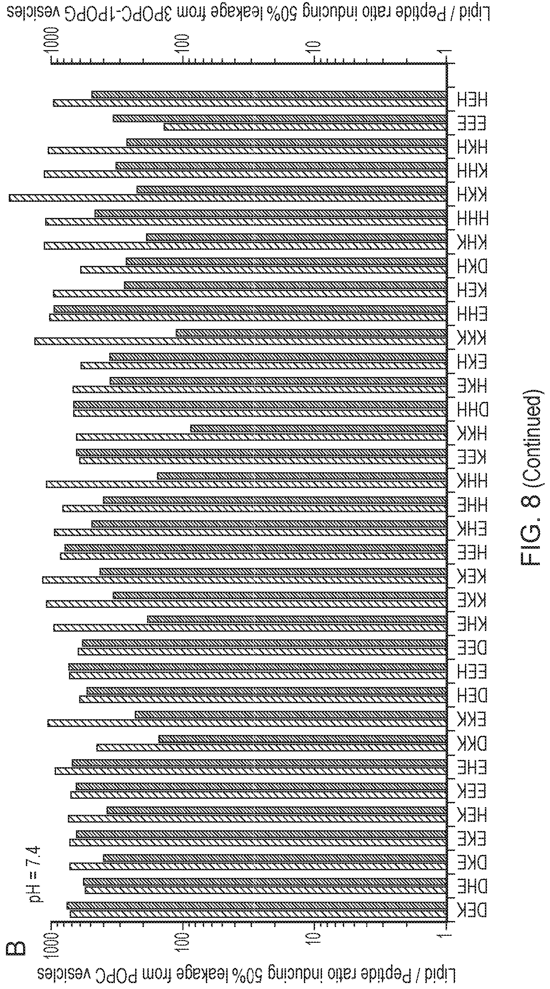

Figure 8 shows the peptide concentration that causes 50% leakage of ANTS/DPX dyes from liposomes. In brief, 0.5 mM POPC vesicles (grey) or POPC:POPG vesicles (ratio 3:1, black) were incubated with peptide concentrations of 0, 0.02, 0.04, 0.08, 0.16, 0.32, 0.64, 1.25, 2.5, 5, 10, and 20 µM in each A) hydrochloric acid-adjusted phosphate buffered saline (1X, pH 4.8) and B) phosphate buffered saline (1X, pH 7.4). The strength of peptide-induced dye leakage is reported as the number of lipids per peptide (a high number signifies a peptide that is more potent at disrupting the lipid membrane).

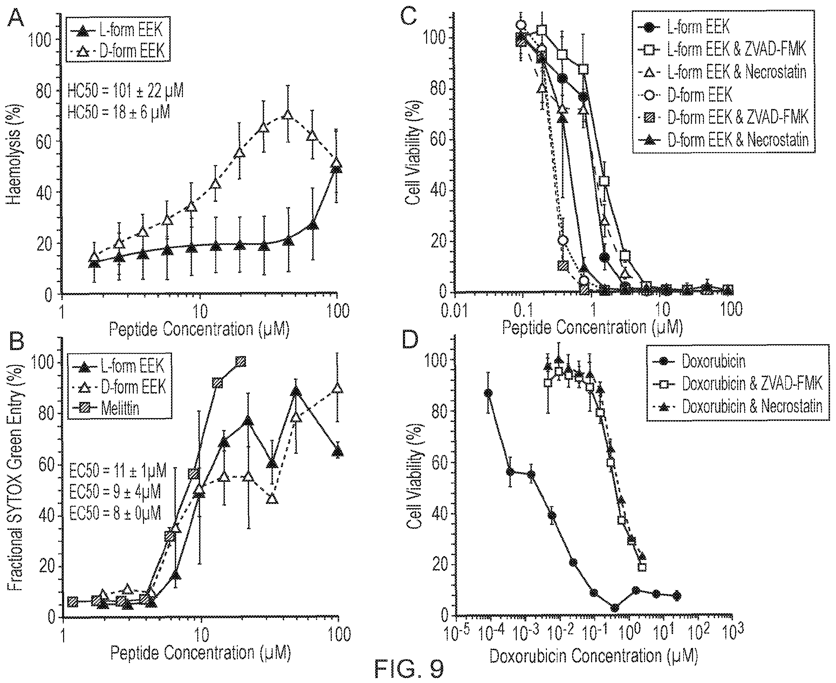

Figure 9 shows the mechanism of action of the leucine-rich ACPs. A) Hemolytic activity of L-form EEK (black triangles) and D-form EEK (grey triangles) against human red blood cells. B) Peptide-induced high-affinity nucleic acid stain (SYTOX green) entry into HeLa cell line with titrated peptide concentrations: L-form EEK (black triangles), D-form EEK (grey triangles), and melittin (squares) as a positive control. C) HMLER-shEcad (human mammary endothelial cancer stem cells) cell viability in the presence of L-form EEK (black circles) and D-form EEK (grey circles) and co-incubated together with necrostatin (inhibitor of necroptosis) and ZVAD- FMK (inhibitor of apoptosis). D) Viability of HMLER-shEcad cells treated with doxorubicin (cirles), and doxorubicin in combination with 5 µM capase inhibitor z-VAD-FMK (square), and doxorubicin with 20 µM necrostatin-1 (triangles)
Figure 10 shows the synthesis strategy for conjugation of the present ACPs with copper-based small molecule anti-cancer drugs.

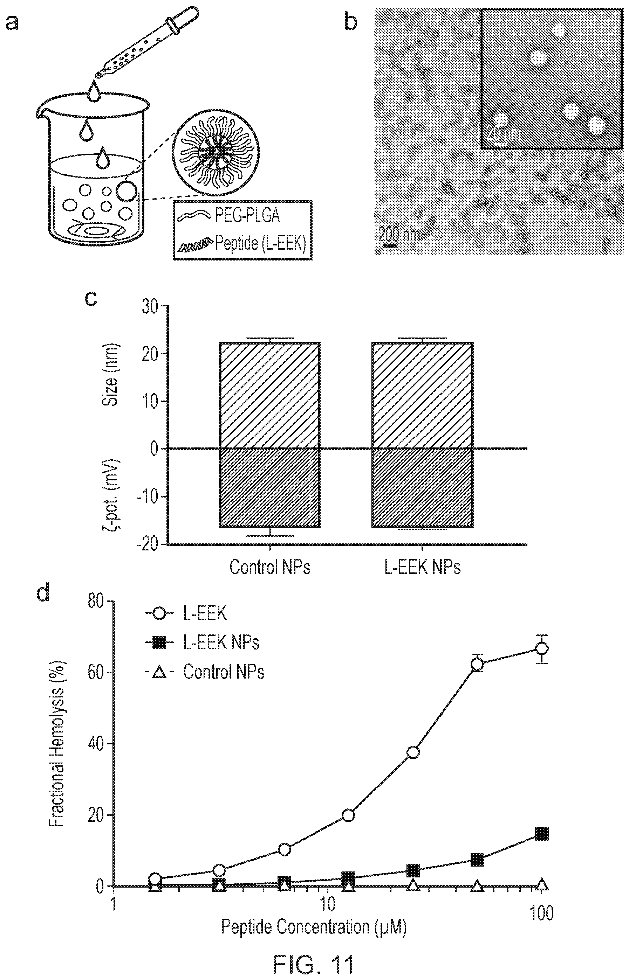

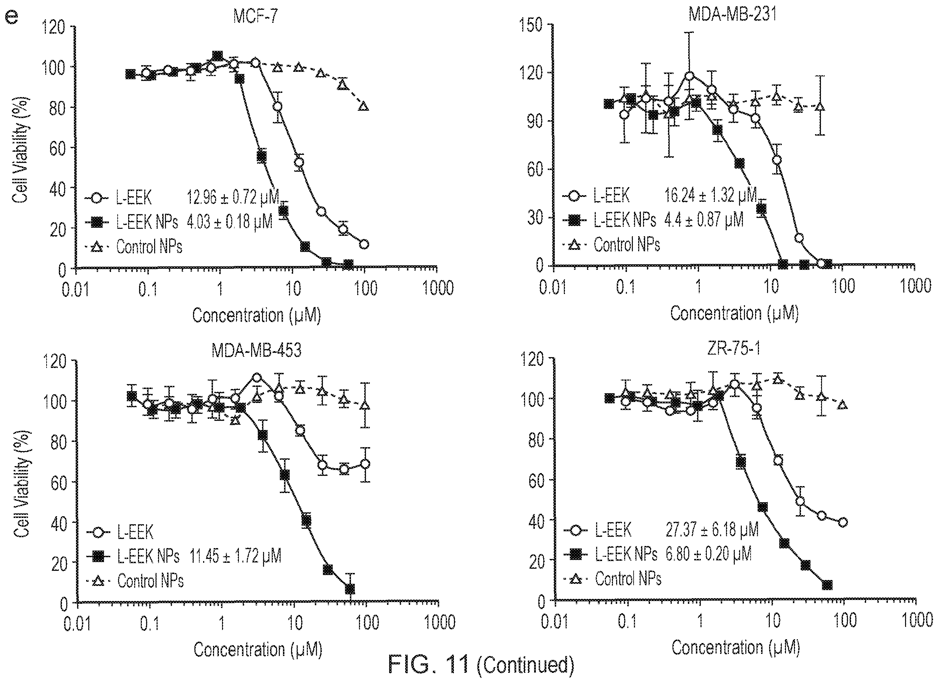

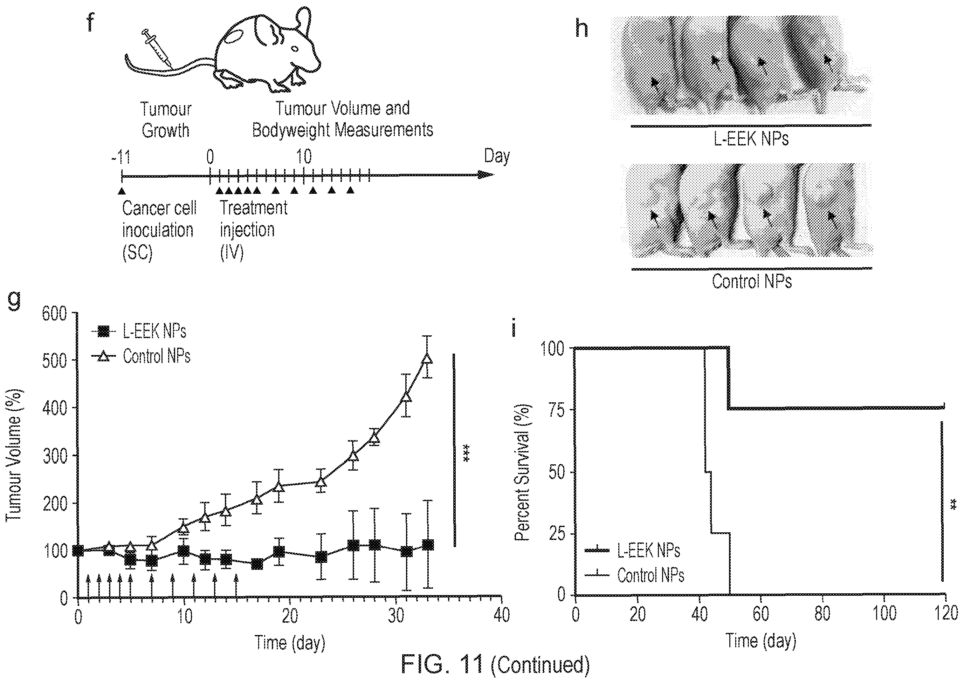

Figure 11 shows the development of EEK nanoparticles (NPs). **a** Schematic illustration of L-EEK NPs and NPs preparation, b Transmission electron microscopy images of L-EEK NPs. Scale bars are 200 nm (black) and 20 nm in the inset (white), **c** Dynamic light scattering characterizations of L-EEK NPs (n = 3) and control NPs without L-EEK cargo, **d** The comparative hemolytic activities of free L-form EEK, L-EEK NPs, and control NPs. **e** Assessment of cell viability by CCK-8 assay with L- EEK, L-EEK NPs, and control NPs treatment against breast cancer cell lines (MCF-7, MDA-MB-231, MDA-MB-453, and ZR-75-1). IC₅₀ values of L-EEK and L-EEK NPs against breast cancer cells are in green and red, respectively, **f** Schematics of the mouse model of MDA-MB-231 triple-negative breast cancer with control nanoparticles and EEK peptide nanoparticles treatment schedule. **G** Efficient inhibition of cancer growth with L-form EEK NPs treatment. Upon establishment of palpable tumours on day 11 following subcutaneous inoculation with MDA-MB-231 (4 x 10⁶ cells), mice were treated with 10 mg/kg/dose of EEK-NPs or equivalent doses of control NPs over a 14-day treatment period. Tumour volumes were monitored. **** p<0.005* (n = 4). **h** Images of MDA-MB-231 tumours on day 33 after the onset of L-EEK NPs and control NPs treatments, i Kaplan-Meier curve of mice survival following tumour inoculation over an observation period of 120 days. Mouse survival is defined as tumour size below 1000 mm³ (n = *4, *p* < 0.05).

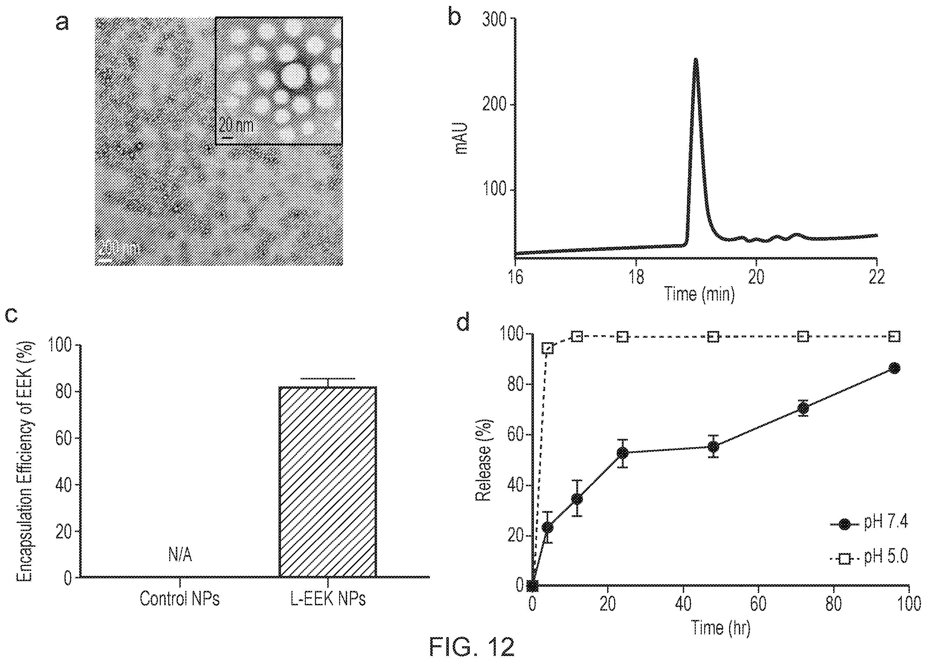

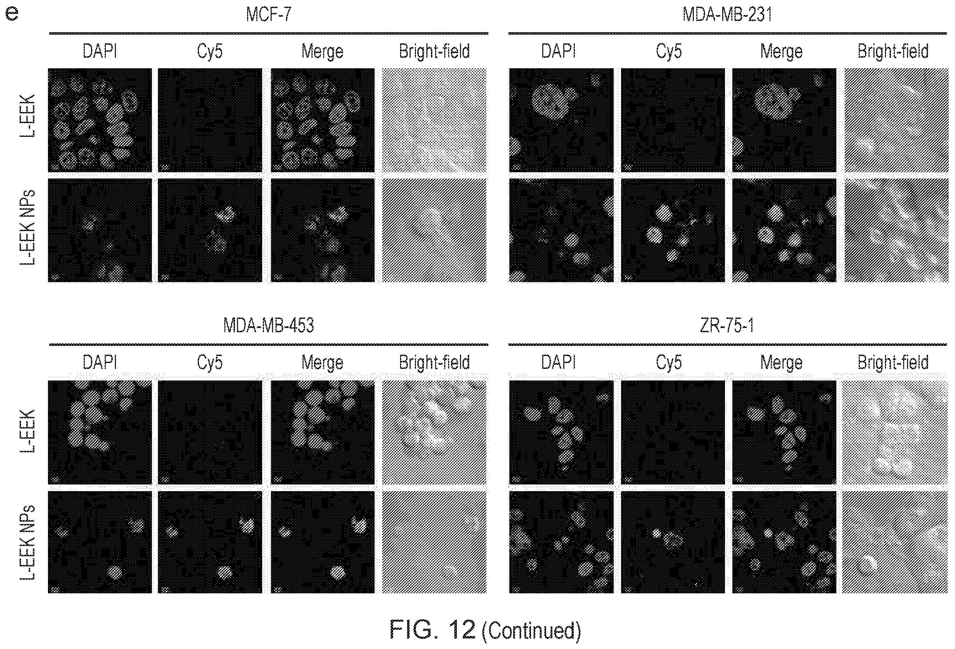

Figure 12 shows the physicochemical properties of L-EEK NPs. **a** TEM images of control NPs. Scale bars = 200 nm and 20 nm in the inset, **b** The chromatograms of L-EEK under HPLC. **c** The encapsulation efficiency of L-EEK in L-EEK NPs. **d** L-EEK release from nanoparticles at pH 7.4 and pH 5.0 at 37 °C. e Cellular uptake of free and nanoparticle-encapsulated Alexa647-labelled L-EEK in breast cancer cell lines.

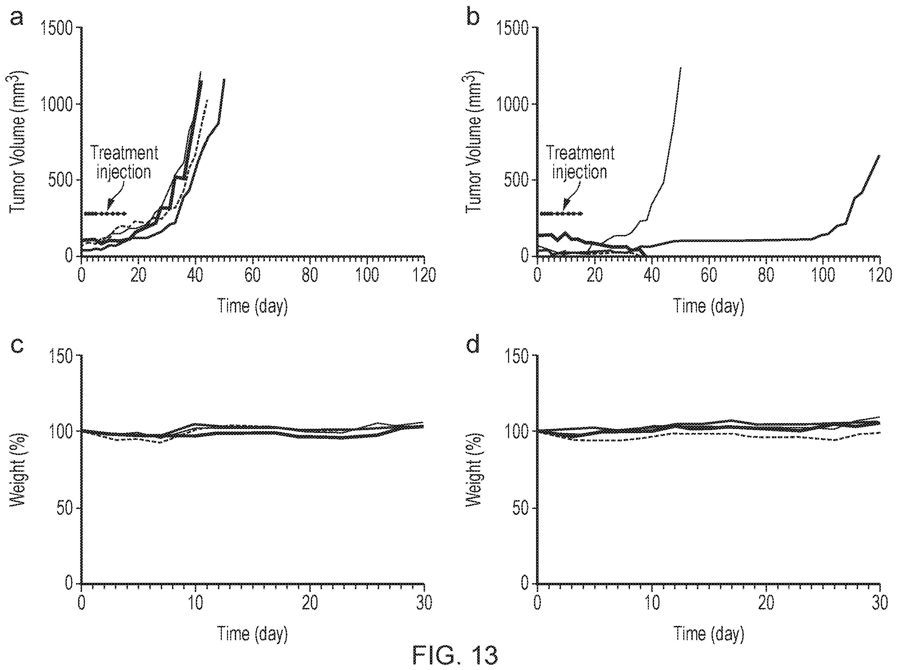

Figure 13 shows anti-cancer efficacy with L-EEK NP treatment, **a** and **c** Individual MDA-MB-231 tumour growth curves and body weight curves for mice treated with control nanoparticles (n = 4). **b** and **d** Individual MDA-MB-231 tumour growth curves and body weight curves for mice treated with L-EEK nanoparticles (n = 4).

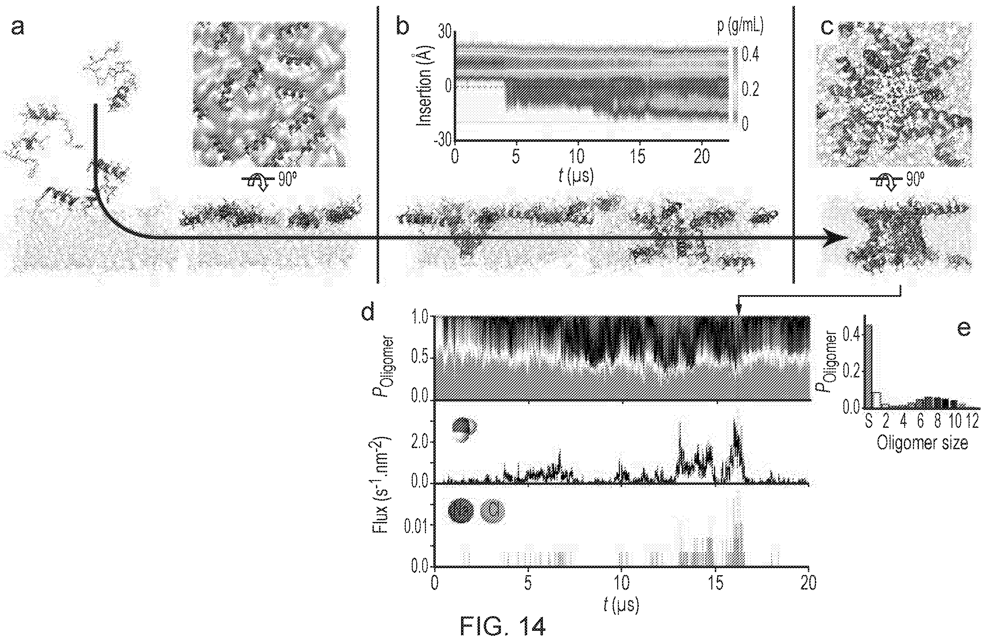

Figure 14 shows that atomic detail ACP membrane pore structures and membrane perforation mechanism. Molecular dynamics simulations reveal the full atomic details of **a,** spontaneous ACP membrane adsorption, **b,** insertion and **c,** pore formation (shown is a large, heterogeneous, fully waterfilled EEK pore). **d,e** Bound peptides form an ensemble of transient pores of 2-16 peptides (top) that conduct both water (middle) and ions (bottom) across the membrane.

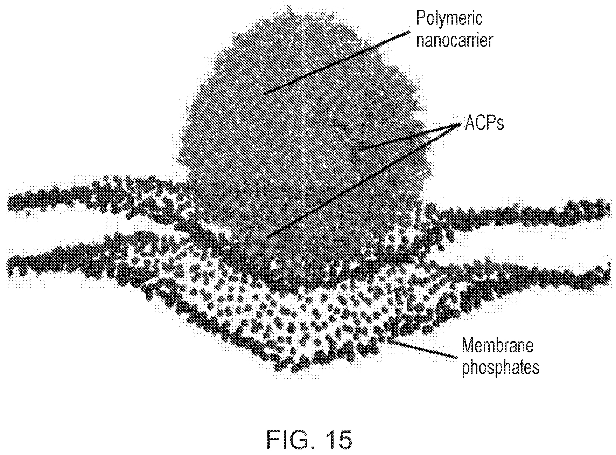

Figure 15 is a molecular simulation of peptide EEK-nanocarrier binding to a cancer cell membrane. ACPs, such as peptide EEK, are freely mobile inside the nanocarrier and enter the target membrane through direct partitioning. The fluid-nature of the polymeric nanocarrier is essential for this process. The size of the nanocarrier, at 20 nm diameter, ensures that the ACPs are delivered locally onto a small ~10 nm diameter area of the target membrane, providing a high local ACP concentration that improves membrane perforation.

### Example 1

### Materials and Methods

### Peptide Synthesis and Purification

Peptides were solid-phase synthesized and purified to 98 % purity. Peptide purity and identity were confirmed by HPLC and ESI mass spectrometry. The N-terminus was a free amine group and the C-terminus was either a free carboxyl group or amidated.

### Liposome Production

The lipids 1-palmitoyl-2-oleoyl-sn-glycero-3-phosphocholine (POPC), 1-palmitoyl-2-oleoyl-snglycero-3-phospho-(1'-rac- glycerol) (POPG) were purchased from Avanti Polar Lipids and dissolved in chloroform. Large unilamellar vesicles (LUVs) were produced by extrusion through 100 nm pore filter using an extruder and filters purchased from Avanti Polar Lipids.

### Cell Lines and Cell Culture Conditions

HMLER (human mammary endothelial cancer cells), HMLER-shEcad (human mammary endothelial cancer stem cells), and MCF-10A (healthy human mammary endothelial) cells were maintained in Mammary Epithelial Cell Growth Medium (MEGM) with supplements and growth factors: bovine pituitary extract (BPE), hydrocortisone, human epidermal growth factor (hEGF), insulin, and gentamicin/amphotericin-B. HEK293T (human embryonic kidney cell), and U2OS (homo sapiens bone osteosarcoma) cells were maintained in Dulbecco's Modified Eagle's Medium (DMEM) with a final concentration of 10 % fetal bovine serum. The cells were grown in T75 flask at 310 K in a humidified atmosphere containing 5 % CO2.

### Cytotoxicity Assay

The colourimetric MTT (3-(4,5-dimethylthiazol-2-yl)-2,5-diphenyltetrazolium bromide) assay was used to determine the toxicity of the anti-cancer peptides and conventional anti-cancer drugs. 5 x 10³ cells were seeded in each well of a 96-well microplate. The cells were incubated overnight. Elevated concentrations of the compounds (0, 0.1, 0.2, 0.4, 0.8, 1.6, 3.1, 6.3, 12.5, 25, 50 and 100 µM) were added and incubated for 72 hr with a total volume 200 µL. The stock solutions of the compounds were prepared as 5 mM solutions in DMSO and diluted using media or in pure water. The final concentration of DMSO in each well was either 0.5 % or 0 % and this amount was present in the untreated control. After 72 hr, 20 µL of a 4 mg/mL solution of MTT in PBS was added to each well, and the plate was incubated for an additional 4 hr. The MEGM/MTT mixture was aspirated and 100 µL of DMSO was added to dissolve the resulting purple formazan crystals. The absorbance of the solutions in each well was read at 550 nm wavelength. Absorbance values were normalized to either DMSO-containing or non DMSO- containing control wells and plotted as concentration of test compound versus % cell viability. IC₅₀ values were interpolated from the resulting dose dependent curves. The reported IC₅₀ values are the average of two independent experiments, each consisting of six replicates per concentration level (overall n = 12). The IC₅₀ values for 36 leucine-rich-based peptides were average of two independent experiments (overall n = 2).

### Tumoursphere Formation and Viability Assay

HMLER-shEcad cells (5 x 10³) were plated in ultralow-attachment 96-well plates (Corning) and incubated in MEGM supplemented with B27 (Invitrogen), 20 ng/mL EGF, and 4 µg/mL heparin (Sigma) for 5 days. Studies were conducted in the absence and presence of anti-cancer peptides, doxorubicin, and salinomycin. Mammospheres treated with anti-cancer peptides, doxorubicin, and salinomycin were counted and imaged using an inverted based reagent, TOX8 (Sigma). After incubation for 16 hr, the fluorescence of the solutions was read at 590 nm (λₑₓ = 560 nm). Viable mammospheres reduce the amount of the oxidized TOX8and concurrently increases the amount of the fluorescent TOX8 intermediate, indicating the degree of mammosphere cytotoxicity caused by the test compound. Fluorescence values were normalized to DMSO-containing or non DMSO-containing controls and plotted as concentration of test compound versus % mammosphere viability. IC₅₀ values were interpolated from the resulting dose dependent curves. The reported IC₅₀ values are the average of two independent experiments, each consisting of two replicates per concentration level (overall n = 4).

### Tryptophan Fluorescent Binding Assay

Peptides (50 µM) and POPC/POPGLUVs (600 µM) were prepared in 10 mM phosphate buffer (pH 7.0). The solutions were incubated and measured after 60 minutes. Excitation was fixed at 280 nm (slit 9 nm) and emission was collected from 300 to 450 nm (slit 9 nm). The spectra were recorded using a Synergy Hl Hybrid Multi-Mode Reader (Figure 3A) and Cytation^{™} 5 Cell Imaging Multi-Mode Reader (Figure 2) from BioTek and were averaged over 3 scans.

### Liposome Leakage Assay

5 mM ANTS (8-aminonaphthalene-1,3,6-trisulfonic acid, disodium salt) and 12.5 mM DPX (p- xylene-bis-pyridinium bromide) were entrapped in 0.1 µm diameter extruded vesicles with lipids. Gel filtration chromatography using a Sephadex G-100 (GE Healthcare Life Sciences Inc) was used to remove external free ANTS/DPX from LUVs with entrapped contents. LUVs were diluted to 0.5 mM and used to measure the leakage activity by addition of aliquots of peptides. Leakage was measured after 3 h incubation. 10% Triton was used as the positive control to measure the maximum leakage of the vesicle. Fluorescence emission spectra were recorded using excitation and emission wavelength of 350 nm and 510 nm for ANTS/DPX using a BioTek Synergy Hl Hybrid Multi-Mode Reader.

### Hemolysis Assay

Peptides were serially diluted in PBS starting at a concentration of 100 µM. The final volume of peptide in each well was 50 µL. To each well, 50 µL of RBCs in PBS at 2 x 10⁸ cells/mL was added. As a positive lysis control, 1% triton was used. The mixtures were incubated at 37 °C for 1 hour, after which they were centrifuged at 1000x g for 5 minutes. After centrifugation, 10 µL of supernatant was transferred to 90 µL of DI H2O in a fresh 96-well plate. The absorbance of released hemoglobin at 410 nm was recorded and the fractional hemolysis was calculated based on the 100% and 0% lysis controls.

### Sytox Green Assay to measure cytotoxicity against Hela cells

Hela cells were grown to confluency in T-75 flasks in complete DMEM (10% FBS). The day prior to cytotoxicity experiments, cells were trypsinized, removed from the flask, and pelleted at 1300 rpm. The trypsin and spent media were discarded and the cells were resuspended in complete DMEM. The cell count was obtained using a cell counter. The cells were then seeded at a density of 10,000 cells/well in a 96-well tissue-culture plate. Next day, in a separate 96-well plate, peptide was serially diluted in complete DMEM (10% with FBS) and 0.1% sytox green starting at a concentration of 100 µM (1st), 67 µM (2nd) which was followed by 2:3 serial dilutions. The final volume of peptide in each well was 100 µL. To perform the cytotoxicity assay, media was removed from the wells and replaced with the peptide/DMEM/sytox green solutions. No peptide and 20 µM MelP5 were used as negative and positive controls, respectively. The plate was read for fluorescence every 5 minutes for an hour with an excitation wavelength of 504 nm and emission wavelength 523 nm. Cytotoxicity was calculated based on the 100% and 0% lysis controls based on the sytox green entered in to the cells due to cell wall destabilization.

### Preparation and Characterisation of Peptide Nanoparticles

In a typical preparation, 0.1 mL of 5 mg/mL L-EEK peptide in methanol was mixed with 1 mL of 25 mg/mL PEG-PLGA in acetonitrile. The mixture was then added into 15 mL of 25 mM Tris buffer (pH8.0), and the solution was stirred with a magnetic stirring bar in a 50 mL glass beaker at 400 rpm for 15 min. Methanol and acetonitrile were then evaporated from the solution completely via nitrogen gas bombardment for 15 min and upon placing the sample solution in vacuum for 1 hr. The nanoparticle solution was then filtered through cellulose acetate syringe filters (pore size 0.45 pm, Sartorius). The filtered L-EEK nanoparticles were washed with 30 kDa centrifugal filter tube (Amicon^{®} Ultra-15 Centrifugal Filter Devices) and concentrated to a final volume of 1 mL. The collected nanoparticles were freshly prepared for the experiments.

### Transmission Electron Microscopy (TEM),

A drop (10 µL) of the L-EEK nanoparticles solution (0.5 mg/mL) was deposited onto a glow-discharged grid. Negative staining was performed with 1 wt.% uranyl acetate for structural examination of L-EEK nanoparticles at room temperature. Negatively stained samples were visualized using the FEI 120 kV Sphera microscope (FEI Tecnai F20).

### Quantification of L-EEK in Nanoparticles.

The L-EEK peptide in nanoparticles was quantified by high-performance liquid chromatography (HPLC). The HPLC analysis was carried out in an Agilent Technologies Series 1100 apparatus (Waldbronn, Germany). The analytical column was an Ascentis Express Cl8 reversed phase column (Supelco, Bellefonte, PA, USA) with a particle size of 5 pm (25 cm x 4.6 mm). The column temperature was maintained at 25°C during the quantification. The mobile phase consisted of phase A (0.1% TFA in acetonitrile) and phase B (0.1% TFA in distilled water). The samples were started with linear gradient elution from 40% to 80% of phase A over 25 min, 80% to 100% of phase A from 25 to 30 min and kept constant for 10 min. Then, the eluent was reversed to the initial composition within 5 min and kept constant for 5 min. The wavelength of detection was set at 220 nm for L-EEK and flow rate was at 0.7 mL/min.

### Measurement of L-EEK release rate from nanoparticles.

L-EEK release from nanoparticles was studied using a dialysis tube with 20k MWCO Slide-A-Lyzer MINI dialysis device (Rockford, IL, USA) in PBS (pH7.4) and in 0.15 M acetate buffer solution (pH5.0). The phosphate buffer and the acetate buffer solution contained 0.3% (v/v) acetic acid and 1.3% (w/v) sodium acetate. The sample was placed into a dialysis tube at 37 °C under gentle stirring. At predetermined time points (1, 4, 8, 12, 24, 48. 72, and 96 hr), the nanoparticle samples were collected and analysed for L-EEK content using HPLC.

### Haemolysis (fresh murine red blood cells).

Fresh murine red blood cells were drawn from BALB/c nude mice, and thoroughly washed in PBS until the supernatant was clear. L-EEK and L-EEK NPs were serially diluted in PBS starting at a concentration of 200 µM. Serial dilution of control NPs was based on the amount of polymer comparing with EEK NPs. The final volume of peptide in each well was 50 µL. To each well, 50 µL of RBCs in PBS at 2x10⁸ cells/mL was added. As a positive lysis control, 1% triton was used. The mixtures were incubated at 37 °C for 1 hr, after which they were centrifuged at 1000xg for 5 minutes. After centrifugation, 10 µL of supernatant was transferred to 90 µL of distilled water in a fresh 96-well plate. The absorbance of released hemoglobin at 410 nm was recorded and the fractional hemolysis was calculated based on the 100% and 0% lysis controls.

### Cell Viability and Cytotoxicity Assays.

The colorimetric Cell Counting Kit-8 (CCK-8) assay was used to determine the cell viability in cell proliferation and cytotoxicity of anti-cancer peptides and conventional anti-cancer drugs. Briefly, 1 x 10⁴ cells were seeded in each well of a 96-well microplate. Free peptides or nanoparticles containing various concentrations of peptides (0, 0.1, 0.2, 0.4, 0.8, 1.6, 3.1, 6.3, 12.5, 25, 50, and 100 µM) were added to the cells and incubated for 72 hr at 37 °C in a humidified atmosphere containing 5% CO2. To each well of the plate was then added 10 µL of CCK-8 solution and incubated for another 4 hr. The absorbance of the solutions in each well was measured at 460 nm. IC₅₀ values were interpolated from the resulting dose dependent curves. The reported IC₅₀ values are the average of two independent experiments, each consisting of six replicates per concentration level (overall n = 3).

### Examination of L-EEK nanoparticle cellular uptake to breast cancer cell lines by confocal microscopy.

Fluorophore-conjugated L-EEK was prepared by incubating L-EEK with Alexa Fluor 647 NHS ester at a 10 to 1 molar ratio in methanol for 72 hr. Following the conjugation, 0.1 mL of 5 mg/mL dye-labelled L-EEK peptide in methanol was mixed with 1 mL of 25 mg/mL PEG- PLGA in acetonitrile. The mixture was then added into 15 mL of 25 mM Tris buffer (pH8.0), and the solution was stirred with a magnetic stirring bar in a 50 mL glass beaker at 400 rpm for 15 min. Methanol and acetonitrile were then evaporated from the solution completely via nitrogen gas bombardment for 15 min and upon placing the sample solution in vacuum for 1 hr. The nanoparticle solution was then filtered through cellulose acetate syringe filters (pore size 0.45 µm, Sartorius). The filtered L-EEK nanoparticles were washed with 100 kDa centrifugal filter tube (Amicon^{®} Ultra-15 Centrifugal Filter Devices) and concentrated to a final volume of 1 mL. The collected nanoparticles were freshly prepared for the experiments. To observe the cellular uptake between cells and L-EEK nanoparticles, fluorescent L-EEK nanoparticles suspended in PBS were incubated with 4 breast cancer cell lines (6 x 10⁴ cells/well). Following 2 hr of incubation in confocal dishes (covered-glass-bottom dish, SPL 200350), cells were washed tree times with PBS to remove unbound free peptides and peptide nanoparticles. The resulting cells were stained with 10 µg/mL DAPI, fixed with 4% paraformaldehyde and examined using a confocal fluorescence microscope (Zeiss LSM 880 with Airyscan).

### Evaluation of the L-form EEK peptide nanoparticles against triple-negative MDA-MB-231 tumour growth in mice.

BALB/c nude mice were inoculated with MDA-MB-231 tumour cells (4 x 10⁶ cells per mouse) subcutaneously on the right flank. The mice were randomly divided into two groups at 11 d post-tumour inoculation. Mice were treated with control nanoparticles (without L-EEK peptide) and peptide nanoparticles with 10 mg/kg of L-EEK peptides by intravenous injection administration. During the treatment period tumour volume and body weight were measured three times per week. Survival end point was set while the tumour volume reached 1000 mm³. The survival curves of individual groups were compared by a log-rank (mantel-cox) test.

### Molecular Dynamics Simulations and analysis

Unbiased all-atom MD simulations were performed and analyzed using GROMACS 2018.3 (www. gromacs.org), Hippo BETA (http://www.biowerkzeug.com), and VMD (http://www.ks.uiuc. edu/Research/vmd/).

Extended peptide structures were generated using Hippo BETA. These initial structures were relaxed via 200 Monte Carlo steps, with water treated implicitly using a Generalized Bom solvent. After relaxation, the peptides were placed in atomic detail peptide/lipid/water systems containing model membranes with 100 mM K and Cl ions using CHARMM-GUI (http ://www.charmm-gui.org/). Protein folding simulations were equilibrated for 10 ns with applying position restraints to the peptide. For pore-forming simulations single peptides were allowed to fold onto the bilayer for ~600 ns. Once a stable surface state had been obtained, subsequently the systems were multiplied 4x4 in the x and y (but not z) directions, resulting in a system with 16 peptides. When starting with peptides from both sides of the membrane, the initial structure had one peptide in the upper and one in the lower leaflet. The large system was then constructed by multiplexing 3x3 to obtain an 18-peptide simulation box. MD simulations were performed with GROMACS 2018.3 using the CHARMM36 force field, in conjunction with the TIP3P water model. Electrostatic interactions were computed using PME, and a cut-off of 10 Å was used for van der Waals interactions. The integration time-step was 2 fs and neighbour lists were updated every 5 steps. All simulations were performed in the NPT ensemble, without any restraints or biasing potentials. Water and the protein were each coupled separately to a heat bath with a time constant τ_{T} = 0.5 ps using velocity rescale temperature coupling. The atmospheric pressure of 1 bar was maintained using weak semiisotropic pressure coupling with compressibility K_{Z} = K_{xy} = 4.6 • 10⁻⁵ bar⁻¹ and time constant τₚ = 1 ps.

### Oligomer population analysis

In order to reveal the most populated pore assemblies during the simulations, a complete list of all oligomers was constructed for each trajectory frame. An oligomer of order n was considered any set of n peptides that are in mutual contact, defined as a heavy-atom (N, C, O) minimum distance of <3.5 Å. Frequently, this definition overcounts the oligomeric state due to numerous transient surface bound (S-state) peptides that are only loosely attached to the transmembrane inserted peptides that make up the core of the oligomer. These S-state peptides frequently change position or drift on and off the stable part of the pore. To focus the analysis on true longer-lived TM pores, a cut-off criterion of 75° was introduced for the tilt angle τ of the peptides. Any peptide with τ ≥75° was considered in the S-state and removed from the oligomeric analysis. This strategy greatly reduced the noise in the oligomeric clustering algorithm by focusing on the true longer-lived pore structures. Population plots of the occupation percentage of oligomer *n* multiplied by its number of peptides *n,* were then constructed. These reveal how much peptide mass was concentrated in which oligomeric state during the simulation time.

### Permutational cluster analysis

All oligomers of the same order n were conformationally clustered using a clustering algorithm with a backbone RMSD similarity cutoff criterion of 4 Å. Since each oligomer could be made up of different peptides - or of the same peptides, but in a different order - the clustering compares one oligomer with all n! permutations of peptide arrangements of another oligomer. Permutations were generated using Heap's algorithm. The final RMSD value of the conformational similarity was considered the lowest RMSD value as obtained from the *n*! permutational comparisons. Clustering results were generally flat, indicating that structures are highly fleeting and dynamical.

### Transmembrane flux

Water and ion flux through membrane pores was calculated by determining the total instantaneous flux through the whole bilayer patch. Two planes orthogonal to the membrane normal were considered at z = -7 Å and z = +7 Å, with all transition events that cross thoe planes counted. The flux was then obtained by dividing the transition counts by the area of the membrane patch and the elapsed time for each trajectory frame. Curves were subsequently smoothed by averaging over 1000 frames.

### Example 2

### Peptide Rationale

Table 1 below comprises 36 peptides of which EEE, KEE, EHE, EEH, DEK and EEK fall within the scope of the present disclosure.

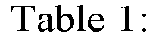

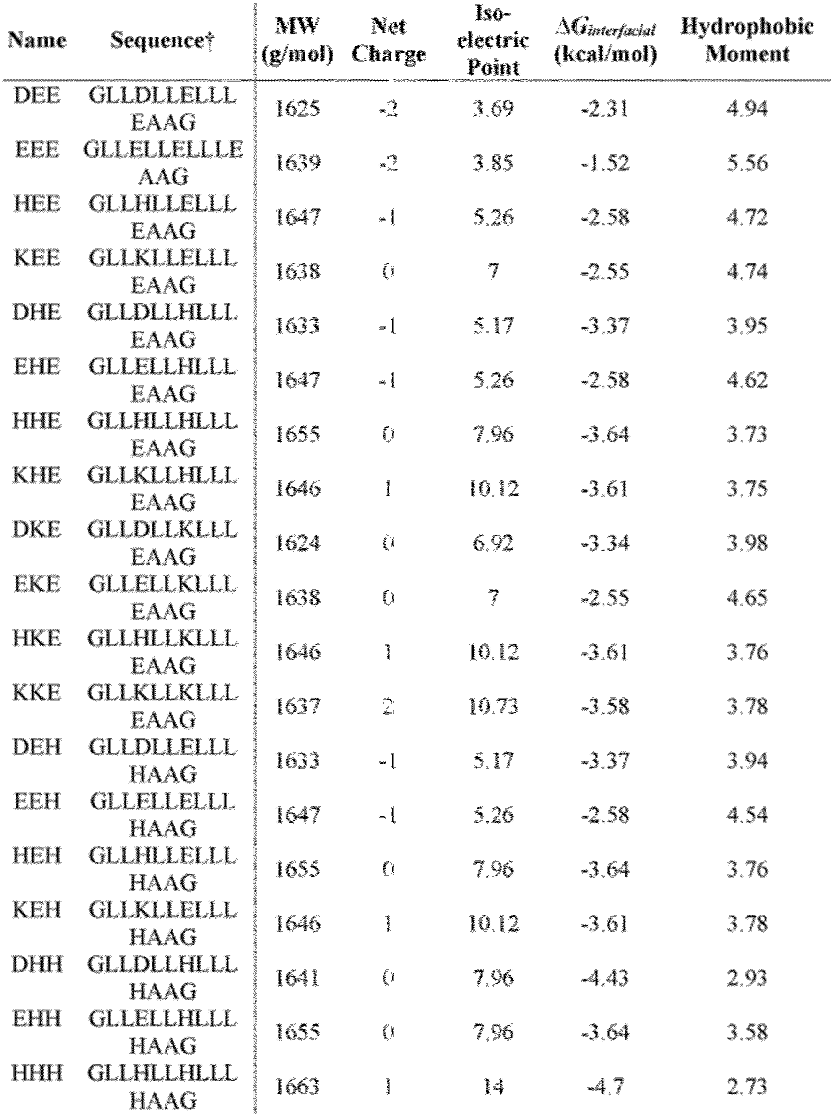

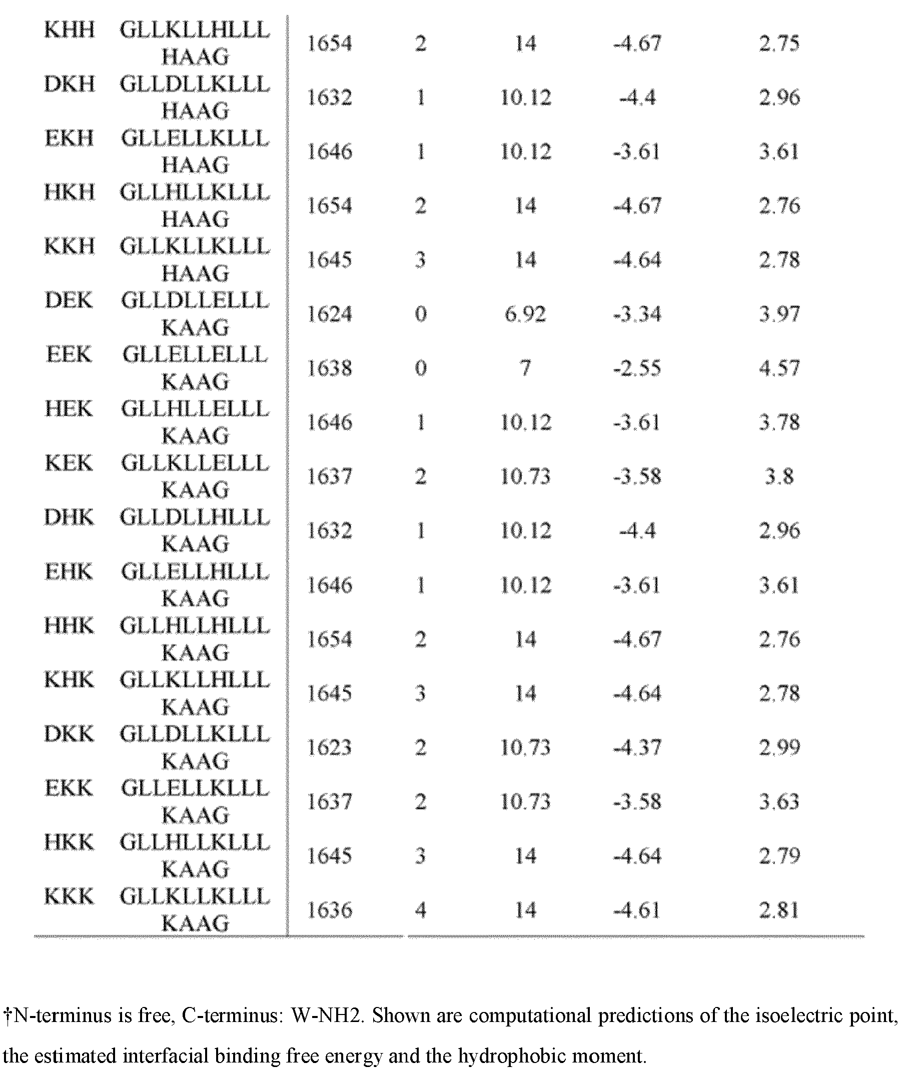

The interfacial binding free energy is a measure of how likely the peptide is to bind to a membrane and the hydrophobic moment is a measure of how evenly the hydrophobic residues are distributed around the surface of the peptide in its helical, membrane inserted, conformation.

An additional tryptophan was introduced at the C-terminus in order to quantify the peptide concentration.

The charged carboxylic C-terminus (-CO2) was also modified to a neutral amide group (-NH2) to further promote membrane penetration. The peptides are designed such that the charged residues are located on the same polar face of the helical structure. Therefore, the charge distribution may affect the peptides' hydrophobic moment, pKa, binding strength onto the cancer cell membrane, and ultimately the structure of the peptide assembly within the cancer cell membrane (Figure 1A). Many pH-dependent peptides with biomedical applications targeting cancer have a pKa ~4.0. This may stem from the slightly more acidic microenvironment of cancer cells, which is due to the Warburg effect. It is therefore believed that the cancer cell membrane can protonate negative amino acids of the present invention, and result in pH-triggered membrane activity (Figure 1B and Table 1).¹⁹⁻²²

All 36 leucine-rich peptide sequences were synthesised as the L-form. *ΔG_{interfacial}* represents the binding free energy of peptide partition between water and the membrane interface. *ΔG_{intefacial}* and hydrophobic moment were estimated using the Wimley-White hydrophobicity scale using the MPEx software. The binding free energy is the energy released upon binding of a peptide to a membrane. At 0 the peptide is 50% in water 50% on the membrane, negative it preferentially inserts, positive it prefers the aqueous phase. The hydrophobic moment is a measure of how the hydrophobic residues are spaced around the helical wheel; a large moment they're all on one side, a low moment they're evenly spaced around. Large moments are better for surface binding (*i.e.* the hydrophobic face dips into the bilayer and the hydrophilic face points to the water).

### Example 3

### Cytotoxicity and Efficacy

The peptides were screened against several different human cell lines and their cytotoxicity were determined. Cell lines utilised include MCF-10A (human breast epithelial cell), HMLER (human breast cancer bulk cell), HMLER-shEcad (human breast cancer stem cell), HEK293T (human embryonic kidney cell), and U2OS (human bone osteosarcoma). It emerged that the peptides are as potent as conventional cancer drugs that can eliminate the cancer cells with low micromolar concentration, and many have high selectivity toward cancer cell lines (Figure 2 and Table 1). Although both doxorubicin and salinomycin also have selectivity for cancerous HMLER over healthy MCF-10A cells, they are both significantly more toxic to HEK293T cells. In addition, both drugs are much less efficient at clearing cancer cells grown as three-dimensional mammospheres, which is considered a far more accurate *in vitro* model for solid tumours at present. The half maximal inhibitory concentrations (IC₅₀) of doxorubicin and salinomycin against two-dimensional HMLER-shEcad are 2.5 ± 0.3 nM and 370 ± 0.5 nM, respectively, however in mammospheres, a more realistic three-dimensional cell culture model that is much more relevant to the *in vivo* condition, these values drop to 43 ± 6 µM and 22 ± 5 µM respectively, a 1,700-fold decrease in activity for doxorubicin and 63 times for salinomycin.

See Table 2 below and Figure 3. In comparison, the selected sequence EEK (GLLELLELLLKAAGW), and its D-form peptide are effective against both two-dimensional as well as three-dimensional mammosphere tumour models, with nano- to low micro-molar activity against two-dimensional cultures of HMLER, HMLER-shEcad, and U2OS cell and 7 - 13 µM activity against mammosphere. See Figures 4 to 6.

All data points were performed in duplicate. The selected D-form peptides, conventional anti-cancer drugs, EEK peptide and 25B2 peptide were repeated six times. The †N-terminus is free, C-terminus: - WNH2.

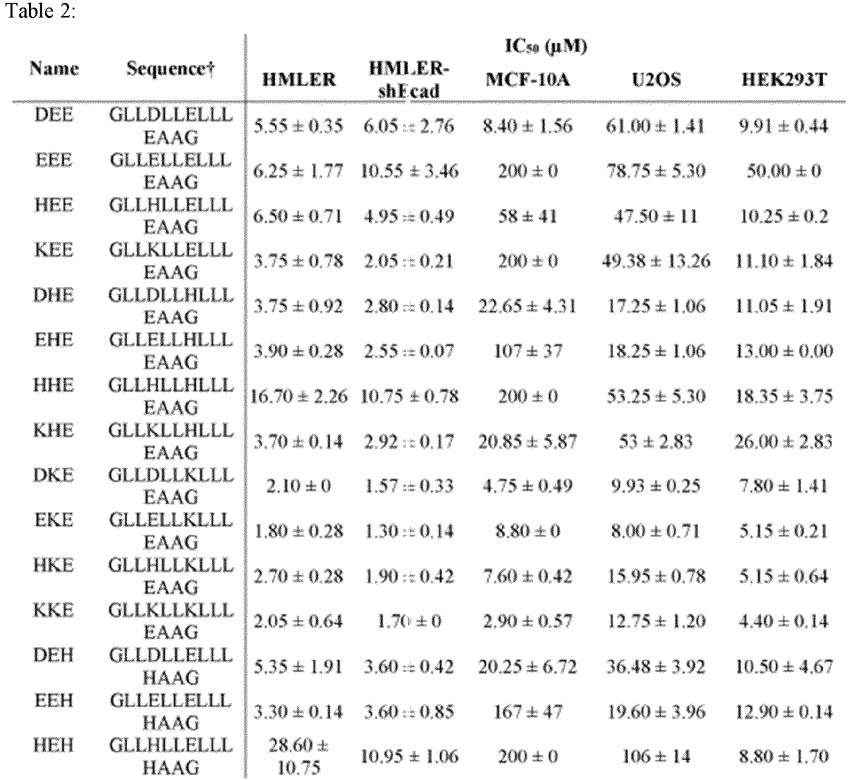

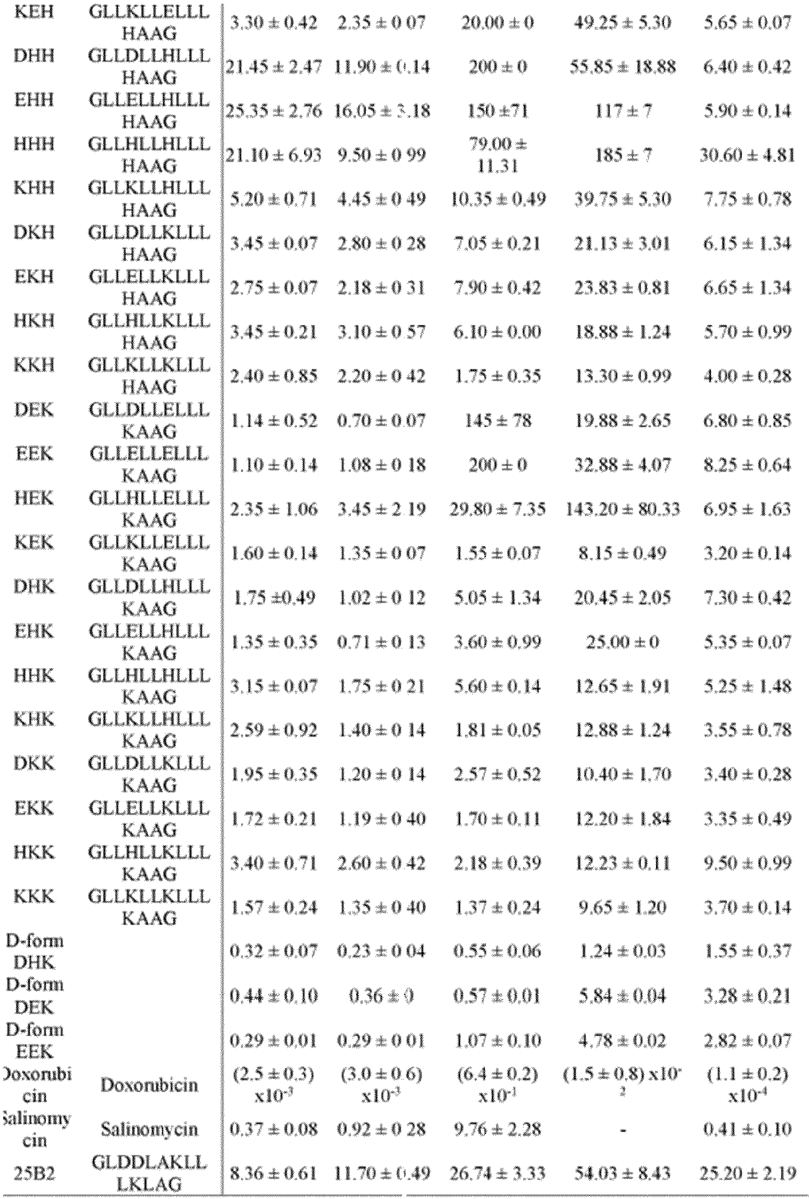

### Example 4

### Tryptophan Binding Assay and Liposome Leakage Assay

The peptides of the present disclosure are mostly neutral or anionic and do not contain many positive charges in the sequence (Table 1). The present inventors identified six sequences (Figure 2 and Table 2) that are highly selective to cancer cell lines and have a negligible effect on MCF-10A (IC₅₀ ≥100 µM) and relatively low cytotoxicity to HEK293T: EEE, KEE, EHE, EEH, DEK, and EEK. Their net charges are between -2 and 0 with a pKa of 3.85-7.96, and their sequences either contain one positive charge (positively charged N-terminus) or two positive charges (one positively charged N-terminus and one lysine at position 4 or 11). Several studies have shown the cancer cell membranes may have a negatively charged membrane surface.^{23,24} Ishikawa *et al.* found that the breast cancer cell line MCF-7, which is similar to HMLER, contains a low amount of negatively charged sialic acid on the membrane surface.²³ This suggests that the anti-cancer activity and cell selectivity of the present leucine-rich peptides cannot solely be explained by electrostatic interactions but may also involve charge distribution due to the Warburg effect in the microenvironment of cancer cells. To confirm this hypothesis, the present inventors performed tryptophan binding assays (See Table 3 below and Figure 7) and ANTS/DPX liposome leakage assay (See Table 4 below and Figure 8) with two different lipid model vesicles (zwitterionic POPC and anionic 3POPC/1POPG mixture) each at pH 7.4 (physiological condition) and pH 4.8 (weak acid).

Table 3 illustrates the lipid concentration-induced 50 % peptide binding onto a liposome. 50 µM peptide was fixed and incubated with titrated lipid (POPC vesicles or 3POPC/1POPG vesicles) at concentrations of 0, 12.5, 25, 50, 100, 250, 500, 1000, 2500, and 5000 µM in phosphate buffered saline (1X, pH 7.4). The lipid concentration that causes 50 % peptide binding was determined using tryptophan fluorescent binding assay and the values are shown as lipid per peptide. †N-terminus is free, C-terminus: -W-NH2.

Table 4 illustrates peptide concentration-induced 50 % ANTS/DPX liposome leakage. 0.5 mM POPC and 3POPC/1POPG vesicles were fixed and incubated with titrated peptide concentration (0, 0.02, 0.04, 0.08, 0.16, 0.32, 0.64, 1.25, 2.5, 5, 10, and 20 µM) each in phosphate buffered saline (1X, pH 7.4) and hydrochloric acid-adjusted phosphate buffered saline (1X, pH 4.8). The values are shown as lipid per peptide. †N-terminus is free, C-terminus: -W-NH2.

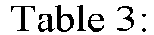

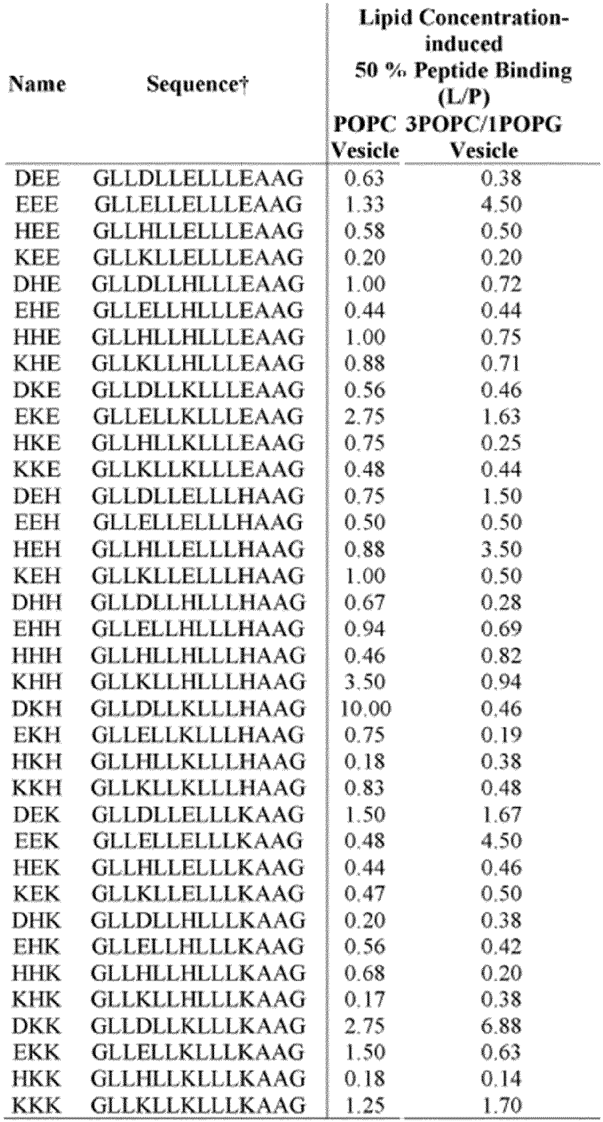

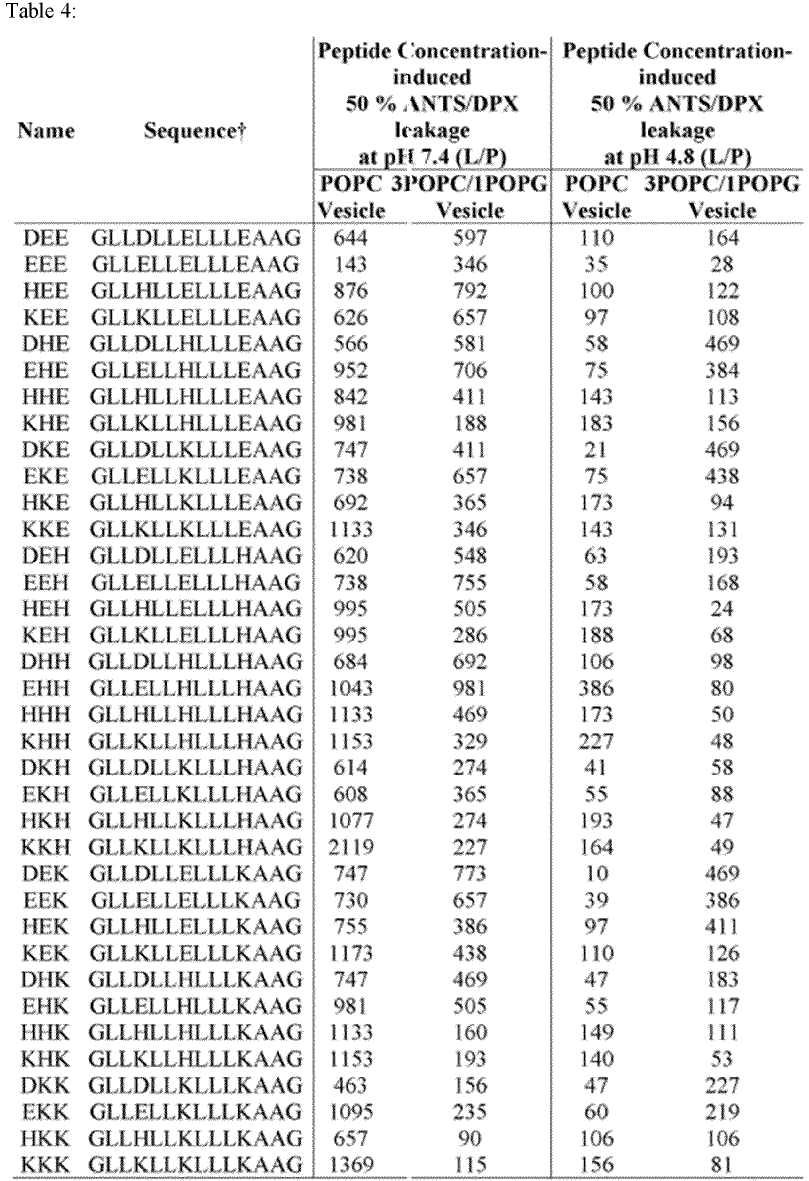

The results show that the cell-selective peptides do not have any significant binding selectivity and peptide-induced liposome leakage between zwitterionic and anionic vesicles at neutral pH, but four (EHE, EEH, DEK, and EEK) out of the six membrane-selective peptides have relatively higher liposome leakage activity from anionic vesicle at pH 4.8. This suggests that these four peptides are environmenttriggered membrane-active peptides that depend on both lipid compositions and pH condition; however, the mechanisms of the other two membrane-selective peptides (EEE and KEE) remain unclear.

### Example 5

### Mechanism of action of the leucine-rich peptides

Figure 9 shows that the L-form of EEK causes minimal lysis below 90 µM concentrations, well below the ~10 µM therapeutic concentration. D-form EEK is more lytic. Comparison of the concentrationdependent entry of SYTOX green, a high-affinity nucleic acid stain, into HeLa cells shows that L-form and D-form EEK behaves similar to the potent pore-forming peptide melittin. Together these results demonstrate selective pore formation of cancer cell-plasma membranes as the as the mechanism of action.

Figure 9C shows that cell viability of HMLER-shEcad cells treated with L or D-form EEK cannot be improved by co-incubation with the necroptosis inhibitor necrostatin, nor by co-incubation with the apoptosis inhibitor z-VAD-FMK, suggesting ACPs trigger necrosis due to pore formation in the plasma membrane. In contrast, Figure 9D shows that the cell viability of HMLER-shEcad cells treated with doxorubicin can be dramatically improved by co-incubation with either z-VAD-FMK or necrostatin.

Together these results suggest selective pore-formation in cancer cell plasma membranes, resulting in necrosis, as the primary mechanisms of ACP anti-cancer activity.

### Example 6

### Nanoparticle Production

To enable intravenous anti-cancer peptide (ACP) delivery and address the solubility, pharmacokinetics, and stability issues that may impede translation of lytic peptide-based therapeutics, ACP-loaded polyethyleneglycol methyl ether polylactide-co-glycolide (PEG-PLGA) nanoparticles (NPs) were prepared, 20 nm in diameter, using an optimized nanoprecipitation method as described above (Preparation and Characterisation of Peptide Nanoparticles). These ultrasmall nanoparticles are advantages over larger carriers in their enhanced ability to penetrate tumours. In addition, we speculate that the diminutive dimension of the 20 nm carriers would enable a more accelerated release profile suitable for the membrane- lytic peptide therapeutic as the peptides need to regain their molecular freedom for membrane perforating actions. L-EEK was selected as the active ACP for NP preparation (L-EEK-NPs) based on its high cancer-specific selectivity in this peptide family.

Following optimisation of the nanoprecipitation protocol (Fig. la), unimodal NPs 21.7±1.4 nm in diameter and with a zeta potential of-16.0±0.6 mV were readily formed (Fig. 1b,c). Control NPs without EEK cargo showed similar physicochemical properties (Fig 2a), suggesting EEK loading is mediated via encapsulation inside the polymeric core rather than surface absorption. HPLC analysis of EEK-NPs showed a high encapsulation efficiency of EEK at 82.3±3.4% (Fig 2b,c), translating to a peptide loading yield of 16.4 µg per mg of polymer. L-EEK-NPs peptide release kinetics are highly pH-sensitive, relinquishing 95.3% of peptides at pH 5.0 in 4 hr, while retaining approximately 50% of the peptide content after 48 hr at the physiological pH of 7.4 (Fig 2d).

Comprised of an acid-labile, biodegradable polymer with a diminutive dimension, the peptide nanoparticles exhibit a highly pH-sensitive release profile critical for the membrane-lytic activity of the encapsulated peptides.

### Example 7

### Nanoparticle Anti-Cancer Efficacy

Cell viability assessed by CCK-8 assay with control NPs, L-EEK peptides, and L-EEK-NPs treatment showed that the NP formulation increased EEK anti-cancer efficacy by a factor of 4 against four different breast cancer cell lines (MCF-7, MDA-MB-231, MDA-MB-453, and ZR-75-1) (Fig. 1e). While enhancing peptide cytotoxicity against cancer cells, NPs were further shown to reduce peptide interaction with red blood cells (Fig. 1d).

### Example 8

### Nanoparticles Can Treat Metastatic Breast Cancer in a Mouse Model

The therapeutic relevance of L-EEK-NPs was assessed in a mouse model of aggressive metastatic MDA-MB-231 triple-negative breast cancer (Fig. If). known for having poor prognosis and limited treatment options. Upon establishment of palpable tumours, mice were treated with either control NPs or L-EEK-NPs over a two-week treatment course. Upon tumour observation following the treatment period, mice in the control group exhibited significant tumour growth in volume (Fig. 1 g,i; Fig. 3a). In contrast, mice that received EEK-NP treatment showed significantly inhibited tumour growth (Fig. 1 g,i; Fig. 3b), with two of the four treated mice showing complete tumour eradication. Notably, both control NP and L-EEK-NP treatments showed negligible body weight loss (Fig. 1h; Fig. 3c,d), attesting to the safety of the anticancer peptide nanoformulation.

### Example 9

### APC Pore Structures and Function

Membrane-perforating peptides typically form transient pores that elude experimental determination with current technology. To reveal the molecular mechanisms underpinning membrane perforation we studied folding-partitioning and pore assembly of EEK using unbiased long-timescale atomic detail molecular dynamics simulations. ACPs rapidly absorb and fold onto the membrane interface (Fig. 14a). Subsequently, on timescales of tens of µs, APCs cooperatively insert and translocate across the lipid bilayer, populating both membrane interfaces (Fig. 14b), and form an ensemble of pores (Fig. 14d). Structure analysis reveals highly heterogeneous pore architectures, with the majority made up of 6-10 peptides that continuously form and disband in the membrane (Fig. 14e). Pores conduct both water and ions (Fig. 14d), and leakage is dominated by larger more stable pores consisting of 10-12 peptides that form large aqueous channels lined with polar and charged side chains (Fig. 14c).

### SEQUENCE DESCRIPTIONS

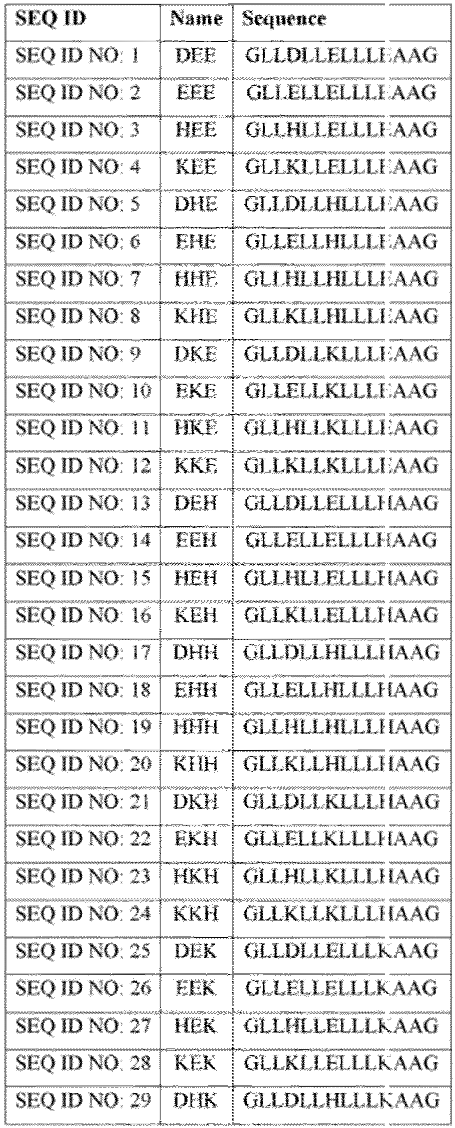

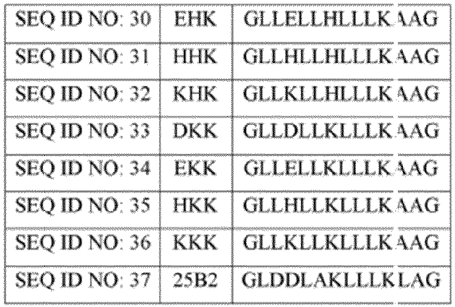

### References

1. Nguyen, L. V.; Vanner, R.; Dirks, P.; Eaves, C. J., Cancer stem cells: an evolving concept. Nat Rev Cancer 2012,12 (2), 133-43.
2. Plaks, V.; Kong, N.; Werb, Z., The cancer stem cell niche: how essential is the niche in regulating sternness of tumor cells? Cell Stem Cell 2015,16 (3), 225-38.
3. Dean, M.; Fojo, T.; Bates, S., Tumour stem cells and drug resistance. Nat Rev Cancer 2005, 5 (4), 275-84.
4. Marx, J., Molecular biology. Cancer's perpetual source? Science 2007, 317 (5841), 1029-31.
5. Kaiser, J., The cancer stem cell gamble. Science 2015, 347 (6219), 226-9.
6. Pattabiraman, D. R.; Weinberg, R. A., Tackling the cancer stem cells - what challenges do they pose? Nat Rev Drug Discov 2014,13 (7), 497-512.
7. Gupta, P. B.; Onder, T. T.; Jiang, G.; Tao, K.; Kuperwasser, C.; Weinberg, R. A.; Lander, E. S., Identification of selective inhibitors of cancer stem cells by high-throughput screening. Cell 2009,138 (4), 645-659.
8. Bao, S.; Wu, Q.; McLendon, R. E.; Hao, Y.; Shi, Q.; Hjelmeland, A. B.; Dewhirst, M. W.; Bigner, D. D.; Rich, J. N., Glioma stem cells promote radioresistance by preferential activation of the DNA damage response. Nature 2006, 444 (7120), 756-60.
9. Papaccio, F.; Paino, F.; Regad, T.; Papaccio, G.; Desiderio, V.; Tirino, V., Concise Review: Cancer Cells, Cancer Stem Cells, and Mesenchymal Stem Cells: Influence in Cancer Development. Stem Cells TranslMed2017, 6 (12), 2115-2125.
10. Izzedine, H.; Perazella, M. A., Anti-cancer Drug-Induced Acute Kidney Injury. Kidney Ini Rep 2017, 2 (4), 504-514.
11. Rosner, M. H.; Perazella, M. A., Acute Kidney Injury in Patients with Cancer. NEngl J Med 2017, 377(5), 500-501.
12. Rosner, M. H.; Capasso, G.; Perazella, M. A., Acute kidney injury and electrolyte disorders in the critically ill patient with cancer. Curr Opin Crit Care 2017, 23 (6), 475-483.
13. Guha S, Ghimire J, Wu E, Wimley WC. Mechanistic Landscape of Membrane- Permeabilizing Peptides. ChemRev. 2019 08;1 19(9):6040-85.
14. Lazzaro BP, Zasloff M, Rolff J. Antimicrobial peptides: Application informed by evolution. Science. 2020 01;368(6490).
15. Chen CH, Lu TK. Development and Challenges of Antimicrobial Peptides for Therapeutic Applications. Antibiot Basel Switz. 2020 Jan 13;9(1).
16. Wimley, W. C.; Hristova, K., Antimicrobial peptides: successes, challenges and unanswered questions. JMembr Biol 2011, 239 (1-2), 27-34.
17. Shai, Y., Mode of action of membrane active antimicrobial peptides. Biopolymers 2002, 66 (4), 236-48.
18. Gaspar, D.; Veiga, A. S.; Castanho, M. A., From antimicrobial to anti-cancer peptides. A review. Front Microbiol 2013, 4, 294.
19. Andreev, O. A.; Engelman, D. M.; Reshetnyak, Y. K., pH-sensitive membrane peptides (pHLIPs) as a novel class of delivery agents. MolMembr Biol 2010, 27 (7), 341-52.
20. Andreev, O. A.; Karabadzhak, A. G.; Weerakkody, D.; Andreev, G. O.; Engelman, D. M.; Reshetnyak, Y. K., pH (low) insertion peptide (pHLIP) inserts across a lipid bilayer as a helix and exits by a different path. Proc Natl Acad Sei USA 2010,107 (9), 4081-6.
21. An, M.; Wijesinghe, D.; Andreev, O. A.; Reshetnyak, Y. K.; Engelman, D. M., pH- (low)-insertion-peptide (pHLIP) translocation of membrane impermeable phalloidin toxin inhibits cancer cell proliferation. Proc Natl Acad Sei USA 2010,107 (47), 20246-50.
22. Wyatt, L. C.; Moshnikova, A.; Crawford, T.; Engelman, D. M.; Andreev, O. A.; Reshetnyak, Y. K., Peptides of pHLIP family for targeted intracellular and extracellular delivery of cargo molecules to tumors. Proc Natl Acad Sei USA 2018,115 (12), E2811-E2818.
23. Ishikawa, K.; Medina, S. H.; Schneider, J. P.; Klar, A. J. S., Glycan Alteration Imparts Cellular Resistance to a Membrane-Lytic Anti-cancer Peptide. Cell Chem Biol 2017, 24 (2), 149-158.
24. Freire, J. M.; Gaspar, D.; Veiga, A. S.; Castanho, M. A., Shifting gear in antimicrobial and anticancer peptides biophysical studies: from vesicles to cells. J Pept Sei 2015, 21 (3), 178-85.

## Claims

1. A nanoparticle comprising one or more peptides having a sequence comprising the motif GLLxLLxLLLxAAG, wherein each x is independently selected from arginine (R), histidine (H), lysine (K), aspartic acid (D) or glutamic acid (E), wherein the nanoparticle has a diameter of 1 to 200 nm and wherein the motif is selected from SEQ ID NO: 2, SEQ ID NO: 4, SEQ ID NO: 6, SEQ ID NO: 14, SEQ ID NO: 25 or SEQ ID NO: 26 and mixtures thereof.

2. A nanoparticle according to claim 1, wherein the one or more peptides have a length of up to 20 amino acids.

3. A nanoparticle according to any preceding claim, wherein the one or more peptides are neutral or anionic.

4. A nanoparticle according to any preceding claim, wherein the one or more peptides are in the L form.

5. A nanoparticle according to any preceding claim, wherein the motif further comprises a C-terminal tryptophan, wherein the charged carboxylic C-terminus is modified to a neutral amide group (W-NH₂).

6. A nanoparticle according to any preceding claim, wherein the nanoparticle has a diameter of from 5 to 100 nm, 10 to 50 nm, or 20 nm.

7. A nanoparticle according to any preceding claim, comprising polyethyleneglycol methyl ether polylactide-co-glycolide (PEG-PLGA).

8. A nanoparticle according to any preceding claim, wherein the peptide forms an alpha helical assembly.

9. A nanoparticle according to any preceding claim, wherein the peptide forms a pore in a cancer cell membrane.

10. A pharmaceutically acceptable composition comprising the nanoparticle of any preceding claim and one or more pharmaceutically acceptable excipients, for use in the treatment of cancer.

11. A pharmaceutically acceptable composition for use according to claim 10, wherein the cancer is breast cancer.

12. A pharmaceutically acceptable composition for use according to claim 10 or 11, wherein the composition is for use in combination with a chemotherapy agent.

13. A pharmaceutically acceptable composition for use according to any of claims 10 to 12, wherein the composition is for administration intravenously.

14. A pharmaceutically acceptable composition for use according to any of claims 10 to 13, wherein the composition is for administration in a dosage ranging from 1 nM to 10,000 nM, preferably from 10 nM to 5,000 nM, more preferably from 100 nM to 500 nM.

## Patentansprüche

1. Ein Nanopartikel, das ein oder mehrere Peptide mit einer Sequenz umfasst, die das Motiv GLLxLLxLLLxAAG umfasst, wobei jedes x unabhängig voneinander aus Arginin (R), Histidin (H), Lysin (K), Asparaginsäure (D) oder Glutaminsäure (E) ausgewählt ist, wobei das Nanopartikel einen Durchmesser von 1 bis 200 nm aufweist und wobei das Motiv aus SEQ ID NO: 2, SEQ ID NO: 4, SEQ ID NO: 6, SEQ ID NO: 14, SEQ ID NO: 25 oder SEQ ID NO: 26 und Mischungen davon ausgewählt ist.

2. Nanopartikel nach Anspruch 1, wobei das eine oder die mehreren Peptide eine Länge von bis zu 20 Aminosäuren aufweisen.

3. Nanopartikel gemäß einem der vorstehenden Ansprüche, wobei das eine oder die mehreren Peptide neutral oder anionisch sind.

4. Nanopartikel gemäß einem der vorstehenden Ansprüche, wobei das eine oder die mehreren Peptide in L-Form vorliegen.

5. Nanopartikel gemäß einem der vorstehenden Ansprüche, wobei das Motiv ferner ein C-terminales Tryptophan (W) umfasst, wobei der geladene Carboxyl-C-Terminus zu einer neutralen Amidgruppe (W-NH₂) modifiziert ist.

6. Nanopartikel gemäß einem der vorstehenden Ansprüche, wobei das Nanopartikel einen Durchmesser von 5 bis 100 nm, 10 bis 50 nm oder 20 nm aufweist.

7. Nanopartikel gemäß einem der vorstehenden Ansprüche, umfassend Polyethylenglykolmethylether-Polylactid-co-glycolid (PEG-PLGA).

8. Nanopartikel gemäß einem der vorstehenden Ansprüche, wobei das Peptid eine Alpha-Helix-Anordnung bildet.

9. Nanopartikel gemäß einem der vorstehenden Ansprüche, wobei das Peptid eine Pore in einer Krebszellmembran bildet.

10. Eine pharmazeutisch akzeptable Zusammensetzung, die das Nanopartikel gemäß einem der vorstehenden Ansprüche und einen oder mehrere pharmazeutisch akzeptable Hilfsstoffe umfasst, zur Verwendung bei der Behandlung von Krebs.

11. Eine pharmazeutisch akzeptable Zusammensetzung zur Verwendung gemäß Anspruch 10, wobei es sich bei dem Krebs um Brustkrebs handelt.

12. Eine pharmazeutisch akzeptable Zusammensetzung zur Verwendung gemäß Anspruch 10 oder 11, wobei die Zusammensetzung zur Verwendung in Kombination mit einem Chemotherapeutikum bestimmt ist.

13. Eine pharmazeutisch akzeptable Zusammensetzung zur Verwendung gemäß einem der Ansprüche 10 bis 12, wobei die Zusammensetzung zur intravenösen Verabreichung bestimmt ist.

14. Eine pharmazeutisch akzeptable Zusammensetzung zur Verwendung gemäß einem der Ansprüche 10 bis 13, wobei die Zusammensetzung zur Verabreichung in einer Dosierung im Bereich von 1 nM bis 10.000 nM, vorzugsweise von 10 nM bis 5.000 nM, noch bevorzugter von 100 nM bis 500 nM, bestimmt ist.

## Revendications

1. Nanoparticule comprenant un ou plusieurs peptides ayant une séquence comprenant le motif GLLxLLxLLLxAAG, dans lequel chaque x est indépendamment choisi parmi l'arginine (R), l'histidine (H), la lysine (K), l'acide aspartique (D) ou l'acide glutamique (E), dans laquelle la nanoparticule a un diamètre de 1 à 200 nm et dans laquelle le motif est choisi parmi SEQ ID NO : 2, SEQ ID NO : 4, SEQ ID NO : 6, SEQ ID NO : 14, SEQ ID NO : 25 ou SEQ ID NO : 26 et leurs mélanges.

2. Nanoparticule selon la revendication 1, dans laquelle le ou les peptides ont une longueur allant jusqu'à 20 acides aminés.

3. Nanoparticule selon l'une quelconque des revendications précédentes, dans laquelle le ou les peptides sont neutres ou anioniques.

4. Nanoparticule selon l'une quelconque des revendications précédentes, dans laquelle le ou les peptides sont sous forme L.

5. Nanoparticule selon l'une quelconque des revendications précédentes, dans laquelle le motif comprend en outre un tryptophane C-terminal (W), dans laquelle l'extrémité C-terminale carboxylique chargée est modifiée en un groupe amide neutre (W-NH₂).

6. Nanoparticule selon l'une quelconque des revendications précédentes, dans laquelle la nanoparticule a un diamètre compris entre 5 et 100 nm, entre 10 et 50 nm, ou de 20 nm.

7. Nanoparticule selon l'une quelconque des revendications précédentes, comprenant du polyéthylèneglycol méthyl éther polylactide-co-glycolide (PEG-PLGA).

8. Nanoparticule selon l'une quelconque des revendications précédentes, dans laquelle le peptide forme un assemblage hélicoïdal alpha.

9. Nanoparticule selon l'une quelconque des revendications précédentes, dans laquelle le peptide forme un pore dans une membrane cellulaire cancéreuse.

10. Composition pharmaceutiquement acceptable comprenant la nanoparticule de l'une quelconque des revendications précédentes et un ou plusieurs excipients pharmaceutiquement acceptables, destinée à être utilisée dans le traitement du cancer.

11. Composition pharmaceutiquement acceptable destinée à être utilisée selon la revendication 10, dans laquelle le cancer est un cancer du sein.

12. Composition pharmaceutiquement acceptable destinée à être utilisée selon la revendication 10 ou 11, dans laquelle la composition est destinée à être utilisée en association avec un agent chimiothérapeutique.

13. Composition pharmaceutiquement acceptable destinée à être utilisée selon l'une quelconque des revendications 10 à 12, dans laquelle la composition est destinée à être administrée par voie intraveineuse.

14. Composition pharmaceutiquement acceptable destinée à être utilisée selon l'une quelconque des revendications 10 à 13, dans laquelle la composition est destinée à être administrée à une dose comprise entre 1 nM et 10 000 nM, de préférence entre 10 nM et 5 000 nM, et plus préférablement entre 100 nM et 500 nM.
